# EUROPEAN PATENT SPECIFICATION

(11) **EP 2 061 433 B1**
(45) Date of publication and mention of the grant of the patent: **16.02.2011**
(21) Application number: 07837876.7
(22) Date of filing: 07.09.2007
(51) Int. Cl.: A61K 9/127, A61K 9/16, A61K 9/50, A61K 9/51, A61K 31/70, A61K 47/26, A61K 47/48, A61K 48/00

(54) **COMPOSITIONS FOR ENHANCING TRANSPORT THROUGH MUCUS**
ZUSAMMENSETZUNGEN ZUR TRANSPORTINTENSIVIERUNG DURCH DIE SCHLEIMHAUT
COMPOSITIONS DESTINÉES À AMÉLIORER LE TRANSPORT À TRAVERS LE MUCUS

(30) Priority: 08.09.2006 US 843282 P
(43) Date of publication of application: 27.05.2009
(73) Proprietor: JOHNS HOPKINS UNIVERSITY, Baltimore, MD 21201 (US)
(72) Inventor: HANES, Justin, Baltimore, MD 21212 (US); LAI, Samuel, K., Baltimore, MD 21211 (US)
(74) Representative: Vossius & Partner
(86) International application number: PCT/US2007/019522
(87) International publication number: WO 2008/030557

(56) References cited:
- WO-A-98/29097
- WO-A-99/01498
- WO-A-02/053189
- WO-A-2005/072710
- WO-A-2006/044660
- AU-A1- 2006 220 411
- US-A1- 2004 258 763
- US-A1- 2005 101 676
- US-A1- 2006 083 781

## Description

### Background

Organs exposed to the external environment, including the lung airways, nasal respiratory tract, gastrointestinal tract, and cervical vaginal tract are protected from entry of foreign particles (including some pathogens and toxins) by a highly viscous and elastic mucus gel. Human mucus has evolved to trap foreign particles sterically and/or by adhesion, and then clear them from the body before they reach the underlying epithelia; particles trapped in mucus can also undergo bacterial or enzymatic degradation. Although clearance rates are anatomically determined, mucus turnover rates in the GI tract are estimated as between 24 and 48 h. In the lungs, clearance rates are dependent on the region of particle deposition; however, normal tracheal mucus velocities, albeit more rapid than mucus velocities in the peripheral lung, range from 1-10 mm/min and turnover times are less than 1h. As a result, the mucus barrier has been cited as a critical bottleneck in the treatment of a variety of diseases.

The primary component of mucus is higher molecular weight mucin glycoproteins, which form numerous covalent and noncovalent bonds with other mucin molecules and various constituents, including DNA, alginate, and hyaluronan. (Hanes et al., Gene therapy in the lung, in Pharmaceutical Inhalation Aerosol Technology, 2d. ed.; Marcel Dekker Inc.: New York, 2003; pp. 489-539). The dense, complex microstructure and high density of hydrophobic and negatively charged domains give rise to a highly viscoelastic and adhesive gel, which significantly impedes the transport rates of large macromolecules and nanoparticles. (Saltzman et al., Biophys. J. 1994, 66, 508-515; Sanders et al., Am. J. Respir. Crit. Care Med. 2002, 162, 1905-1911; Olmsted et al., Biophys. J. 2001 81, 1930-1937). To overcome the mucus barrier, drug carriers must quickly traverse mucus layers that are up to a few hundred microns thick in order to reach the underlying epithelia and avoid clearance mechanisms. Difficulty in drug-carrier particle transport through mucus is thought to be due to a very small average mesh pore size (estimates

range from 5-10 nm to no larger than 200 nm) of highly elastic human mucus, and to its strongly adhesive nature (Olmsted, S.S., J.L. Padgett, A.I. Yudin, K.J. Whaley, T.R. Moench, and R.A. Cone, Diffusion of macromolecules and virus-like particles in human cervical mucus. Biophysical Journal, 2001. 81(4): p. 1930-1937). Cone and coworkers recently showed that standard latex (i.e., polystyrene) polymer particles as small as 59 nm in diameter are completely immobile in mucus since they firmly adhere to mucin fibers, causing it to assemble into mucus strands, or "bundles". These observations have suggested that efficient transport of synthetic polymer nanoparticles, especially those larger than 59 nm, through human mucus barriers is a daunting task.

The international publication WO2005072710 discloses polymeric or liposomal particles with the surface - altering agent polyethylene glycol (PEG) in order to enhance the transport through the human mucus. The particles comprise a pharmaceutically acceptable polymer core and a bioactive agent disposed on the surface. The particles can be comprised in an inhaler. For gene therapy, the particle is a vector or plasmid.

The document US2006083781 relates to aqueous solution containing functionalized polymer, also referred to as surface functional moieties, which form on nanoparticles a hydrophilic surface functional layer. The polymer can be selected from polyethylene glycol (PEG). The particles also comprise bioactive agents.

The disclosure in US2005101676 describes examples of delivery vehicles suitable for the GI or urogenital mucosa include biodegradable microparticles. The particle comprises mucus transport enhancer moieties such as dextrins (cyclodextrin).

The international publication WO9901498 describes the use of a PEG - chitosan conjugate to enhance the systemic uptake of a drug across a mucosal surface. The liposomes, microparticles, microcapsules and nanoparticles carry a conjugate on the surface of which 1-50% on a weight basis is PEG.

US2004258763 describes core particles coated with a surface modifying agent such as PEG.

WO9829097 teaches compositions for increased bioavailability through mucosal delivery comprising powdery particles and a surface active agent such as PEG-monostearate and sodium lauryl sulfate.

WO02053189 describes micro-particle carrier which are coated with a polymer on the surface.

WO2006 044660 discloses solid lipid nanoparticles having an exterior layer of functionalised polymers such as PEG and/or surfactant and/or biologically active agent.

### Summary of the Invention

The present invention relates in part to the finding that surface-altering agents can be used to decrease the mucoadhesion of a substance and increase its mobility in mucus. Thus, in one aspect the invention provides a particle modified with one or more surface-altering moieties that facilitate passage of the particle through mucus wherein (a) the mass of the surface-altering moiety makes up at least 1/3400 of the mass of the particle, and/or (b) the surface-altering moiety is present on the outer surface at a density of greater than 0.01 units per nanometer squared. Such particles, e.g., nanoparticles or microparticles, have a higher concentration of surface moieties than has been previously achieved, leading to the unexpected property of rapid diffusion through mucus. The present invention further comprises a pharmaceutical composition of such particles with carriers, an inhaler comprising such particles.

The invention further concerns a pharmaceutical preparation suitable for inhalation, injection or topical administration to a mucus membrane, comprising a bioactive agent associated with a surface-altering moiety wherein (a) the mass of the surface-altering moiety makes up at least 1/3400 of the mass of the bioactive agent and/or (b) the surface-altering moiety is present on the outer surface of the bioactive agent at a density of greater than 0.01 units per nanometer squared.

The invention is also concerned with an enveloped virus comprising a surface-altering moiety, wherein said virus diffuses through human cervicovaginal mucus at a diffusivity at a time scale of 1 second that is more than 20-fold greater than the diffusivity at which a corresponding virus lacking the surface-altering moiety diffuses through human cervicovaginal mucus, and wherein (a) the mass of the surface-altering moiety makes up at least 1/3400 of the mass of the virus and/or (b) the surface-altering moiety is present on the outer surface of the virus at a density of greater than 0.01 units per nanometer squared.

In certain embodiment, the present invention provides surface-altered particles and methods of making and using them. Suitable particles include polymeric, liposomal, metal, metal oxide, viral, or quantum dot particles, or any combination thereof, that are capable of efficiently traversing mucus layers coating mucosal surfaces. In certain embodiments, such particles may comprise one or more bioactive agents, which may be disposed on the surface of the particle or in the interior of the particle, e.g., encapsulated in a vehicle, such as a polymer. In certain embodiments, the one or more bioactive agents are covalently or non-covalently associated with the particle. Suitable polymeric particles may comprise a pharmaceutically acceptable polymer core and a surface-altering agent. Liposomal particles generally comprise a liposome core and a surface-altering agent. Particles may comprise one or more bioactive agents and/or imaging agents. The surface-altering agent may comprise one or more chemical entities, or may, for example, be incorporated (e.g., physically, as a mixture, or covalently, such as a block copolymer or a covalently modified polymer) into the polymer vehicle. The particles may also comprise one or more targeting moieties.

Certain embodiments provide particles that are, on average, greater than 1, 2, 5, 10, 20, 50, 55, 59, 75, 100, 150, 200, 300, 400, 500, 750, 1000, 2000, or 5000 nm in diameter, or that have a diameter intermediate between any of these values. In certain embodiments, the particles have an average diameter less than 10,000 nm or 50,000 nm. Certain embodiments provide particles that are, on average, larger than the largest estimated mucal pore size, which is 100 nm. In certain embodiments, the diameter is the physical diameter. In such embodiments, the diameter of a nonspherical particle is the largest linear distance between two points on the surface of the particle. In certain embodiments, the diameter is the hydrodynamic diameter. In certain embodiments, the diameter of a nonspherical particle is the hydrodynamic diameter.

In certain embodiments, the present invention provides a particle that can diffuse through a mucosal barrier at a greater rate or diffusivity than a corresponding particle, e.g., unmodified polystyrene particles. A particle of the invention may pass through a mucosal barrier at a rate or diffusivity that is at least 10, 20, 30, 50, 100, 200, 500, 1000, 2000, 5000, 10000- or greater fold higher than a corresponding particle. In addition, a particle of the invention may pass through a mucosal barrier with a geometric mean squared displacement that is at least 10, 20, 30, 50, 100, 200, 500, 1000, 2000, 5000, 10000- or greater fold higher than a corresponding particle at a time scale of 1 s. The corresponding particle may comprise a carboxyl-modified polystyrene particle, an amine-modified polystyrene particle, or a sulfate-aldehyde modified polystyrene particle. Such a carboxyl-modified particle preferably has carboxyl groups present at a density of 1.77 to 6.69 carboxyls per nm². For the purposes of such comparison, The corresponding particle may be approximately the same size, shape, and/or density as the particle of the invention.

In certain embodiments, the present invention provides particles that can diffuse through a mucosal barrier at rate approaching the rate or diffusivity at which said particles can diffuse through water. A particle of the invention may pass through a mucosal barrier at a rate or diffusivity that is at least 1/1000, 1/600, 1/500, 1/200, 1/100, 1/50, 1/20, 1/10, 1/5, 1/2, or I times the rate of the particle in water under identical conditions.

In certain embodiments, the present invention provides particles comprising a surface-altering agent at a given density. A particle of the invention may comprise a surface-altering agent at a density of at least 0.001, 0.002, 0.005, 0.01, 0.02, 0.05, 0.1, 0.2, 0.5, 1, 2, 5, 10, 20, 50, or 100 units per nm².

In certain embodiments, the present invention provides particles that travel through mucus, such as human cervicovaginal mucus, at certain absolute diffusivities. For example, the particles of the present invention may travel at diffusivities of at least 1x10⁻⁴, 2 x10⁻⁴, 5x10⁻⁴, 1 x10⁻³, 2x10⁻³ 5x10⁻³, 1x10⁻² , 2 x10⁻², 4 x10², 5x10⁻², 6x10⁻², 8x10⁻² 1x10⁻¹ , 2x10⁻¹,5x10⁻¹ ,1, or 2 µm²/s at a time scale of I s.

In certain embodiments, the present invention provides particles comprising a surface-altering agent wherein the mass of the surface-altering moiety makes up at least 1/10,000, 1/5000, 1/3400, 1/2000, 1/1000, 1/500, 1/200, 1/100, 1/50, 1/20, 1/5, 1/2, or 9/10 of the mass of the particle.

In certain embodiments, the present invention provides particles comprising a surface-altering agent that inhibits the adsorption of fluorescently labeled avidin, wherein the particle adsorbs less than 99%, 95%, 90%, 70%, 50%, 40%, 30%, 20%, 15%, 10%, 8%, 6%, 5%, 4%, 3%, 2%, 1%, 0.5%, 0.1%, 0.05%, or 0.01% of the amount of fluorescently labeled avidin that is adsorbed by a corresponding particle lacking the surface-altering agent, as calculated by average maximum fluorescent intensity.

In certain embodiments, the present invention provides particles comprising a surface-altering agent that affects the zeta-potential of the particle, wherein the zeta potential of said particle is between -100 mV and 10 mV, between -50 mV and 10 mV, between -25 mV and 10 mV, between -20 mV and 5 mV, between -10 mV and 10 mV, between -10 mV and 5 mV, between -5 mV and 5 mV, or even between -2 mV and 2 mV. The invention further comprises said particle wherein the zeta potential of said particle is less than 5 mV. The invention further comprises said particle wherein the zeta potential of said particle is less than 10 mV.

In certain embodiments, the present invention provides the particles of any preceding paragraph, wherein the exponent of a power law fit of the mean squared displacement of the particle population as a function of time scales from 0.067 s to 3.0 s exceeds 0.1, 0.2, 0.5, 0.8, or 0.9.

An additional aspect of the invention relates to a pharmaceutical composition comprising a particle of the invention, e.g., one or more particles as described herein and/or having one or more of the qualities described above. In certain embodiments, the pharmaceutical composition is adapted for topical delivery to a mucosal tissue in a patient. Said pharmaceutical composition may be delivered to a mucosal surface in a patient, may pass through a mucosal barrier in the patient, and/or may exhibit prolonged residence time on a mucus-coated tissue, e.g., due to reduced mucoadhesion. In certain embodiments, polymeric particles described herein, with or without a bioactive agent, can be administered to a patient, e.g., to treat, inhibit, or prevent a viral infection.

In certain embodiments, the invention provides a composition comprising a plurality of particles, wherein at least 1%, 2%, 5%, 10%, 15%, 20%, 30%, 40%, 50%, 70%, 90%, 95%, or even at least 99% of the total particles in the composition have one or more of the characteristics described in the preceding paragraphs. In addition, the invention provides a composition comprising a mixture of two or more types of particles, e.g., one of which types comprises particles that have one or more of the characteristics described in the preceding paragraphs.

In one aspect, a particle comprises a pharmaceutically acceptable polymer core and a surface-altering agent that is embedded or enmeshed in the particle's surface or that is disposed (e.g., by coating, adsorption, covalent linkage, or other process) on the surface of the particle. The surface-altering agent may be a bioactive agent itself. For example, in certain embodiments, a particle may comprise a pharmaceutically acceptable polymer and a nucleic acid coating the surface of the particle. In such embodiments, the nucleic acid molecule may alter the surface of the particle and make it mucus-resistant. In certain other embodiments, a particle comprises a pharmaceutically acceptable polymer and a protein (e.g., serum albumin) disposed on the surface of the particle. The protein may alter the surface of the particle and make it mucus-resistant.

In any of the above embodiments, the particle may comprise a therapeutic agent or an imaging agent, e.g., that may include a diagnostic agent and/or a detectable label. For example, a nucleic acid or protein included in the particle may comprise an imaging agent itself, e.g., a detectable label can be attached to the DNA or the protein. Alternatively, the particle may comprise an imaging agent that is separate from the nucleic acid or the protein, e.g., encapsulated in the core or disposed on or coupled to the surface. Additionally, the particle may comprise one or more targeting moieties or molecules coupled to the particle and/or the protein or nucleic acid, and the targeting moiety can help deliver the nucleic acid, the protein, and/or the therapeutic, imaging, and/or diagnostic agent to a targeted location in a patient.

In certain embodiments, a particle comprises a pharmaceutically acceptable polymer core, a bioactive agent (e.g., a drug or medicament) encapsulated in the core, and a surface-altering agent that is embedded or enmeshed in the particle's surface, or disposed (e.g., by coating, adsorption, covalent linkage, or other process) on the surface of the particle and that alters the surface of the particle, e.g., to make it able to diffuse rapidly through mucus. The particle may comprise an imaging agent, e.g., a diagnostic agent and/or a detectable label. The encapsulated bioactive agent may be or comprise an imaging agent itself, e.g., a detectable label may be attached to a therapeutic agent. Alternatively, the particle may comprise an imaging agent that is separate from the bioactive agent. Additionally, the particle may comprise a targeting moiety or molecule coupled to the particle, and the targeting moiety can help deliver the bioactive agent and/or the imaging agent to a desirable location in a patient.

In one aspect, a particle comprises a core having one more more bioactive agents (e.g., a drug or medicament) and a surface-altering agent that is embedded or enmeshed in the particle's surface or that is disposed (e.g., by coating, adsorption, covalent linkage, or other process) on the surface of the particle. The surface-altering agent may be a bioactive agent itself.

Alternatively, a particle may comprise a pharmaceutically acceptable polymer core, a surface-altering agent, e.g., a surfactant, that is embedded or enmeshed in the particle's surface, or disposed (e.g., by coating, adsorption, covalent linkage, or other process) on the surface of the particle and that alters the surface of the particle, such as by making it mucus-resistant, and a bioactive agent disposed on the polymeric particle. The bioactive agent may be coated or otherwise disposed on the surface of the particle, or be coupled to the particle, e.g., by covalent linkage, complexation, or other process. In certain such embodiments, the surface-altering agent is selected to promote adhesion or complexation of the bioactive agent to the surface of the particle. In such embodiments, the surface-altering agent and/or the bioactive agent may contribute to rapid diffusibility through mucus of the modified particles. The particles may comprise an imaging agent, such as a diagnostic agent and/or a detectable label. The bioactive agent coated or disposed on the surface of the particle or coupled to the particle may be or comprise an imaging agent itself, e.g., a detectable label can be attached to a therapeutic agent. Alternatively, the particle may comprise an imaging agent that is separate from the bioactive agent, e.g., encapsulated in the core or disposed on or coupled to its surface. Additionally, the particle may comprise a targeting moiety or molecule coupled to the particle, and the targeting moiety can help deliver the bioactive agent and/or the imaging agent to a targeted location in a patient.

The present invention also provides a particle, comprising a polymer having regions of polyethylene glycol or its derivatives that are presented on the surface of the particle. The particle may optionally comprise an additional surface-altering agent. The particle may further comprise a bioactive agent and/or a targeting moiety.

Bioactive agents according to the invention include but are not limited to a nucleic acid, DNA (e.g., a gene therapy vector or plasmid), an RNA (e.g., an mRNA, the transcript of an RNAi construct, or an siRNA), a small molecule, a peptidomimetic, a protein, peptide, lipid, surfactant and combinations thereof.

The surface-altering agent may alter the charge or increase the hydrophilicity of the particle, or otherwise promote motility through mucus. The surface-altering agent may enhance the average rate at which the particles, or a fraction of the particles, move in or through mucus. Examples of suitable surface-altering agents include but are not limited to anionic protein (e.g., serum albumin), nucleic acids, surfactants such as cationic surfactants (e.g., dimethyldioctadecyl-ammonium bromide), sugars or sugar derivatives (e.g., cyclodextrin), polyethylene glycol, mucolytic agents, or other non-mucoadhesive agents. A preferred embodiment comprises polyethylene glycol covalently linked to the particle core. Certain agents, e.g., cyclodextrin, may form inclusion complexes with other molecules and can be used to form attachments to additional moieties and facilitate the functionalization of the particle surface and/or the attached molecules or moieties. Examples of suitable carbohydrate surface-altering agents include agar, agarose, alginic acid, amylopectin, amylose, beta-glucan, callose, carrageenan, cellodextrins, cellulin, cellulose, chitin, chitosan, chrysolaminarin, curdlan, cyclodextrin, dextrin, ficoll, fructan, fucoidan, galactomannan, gellan gum, glucan, glucomannan, glycocalyx, glycogen, hemicellulose, hydroxyethyl starch, kefiran, laminarin, mucilage, glycosaminoglycan, natural gum, paramylon, pectin, polysaccharide peptide, schizophyllan, sialyl lewis x, starch, starch gelatinization, sugammadex, xanthan gum, and xyloglucan, as well as fragments and derivatives of such carbohydrates.

The particles of the invention have many applications. In particular, they are well-suited for making pharmaceutical compositions, particularly those for which the route of administration involves the particles passing through a mucosal barrier. For example, the particles are particularly suitable for making pharmaceutical compositions to be formulated as nasal spray, such that the pharmaceutical compositions can be delivered across a nasal mucus layer. In addition, the particles are particularly suitable for making pharmaceutical compositions to be formulated as an inhaler, such that the pharmaceutical compositions can be delivered across a pulmonary mucus layer. Similarly, the particles are particularly suitable for making pharmaceutical compositions for delivery via gastrointestinal, respiratory, rectal, and/or vaginal tissues.

A pharmaceutically acceptable polymer may be a poly(D,L-lactic-co-glycolic) acid, polyethylenimine, dioleyltrimethyammoniumpropane/dioleyl-sn-glycerolphosphoethanolamine, polysebacic anhydride, or other polymer formed from clinically acceptable or approved monomers. Examples of clinically approved monomers include but are not limited to monomers of sebacic acid and 3-bis(carboxyphenoxy)propane. Other polymers or copolymers described herein can also be employed to make the polymeric particles of the invention.

In certain embodiments, a bioactive agent is a therapeutic agent or an imaging agent (e.g., a diagnostic agent). Examples of therapeutic agents include but are not limited to a nucleic acid, a nucleic acid analog, a small molecule, a peptidomimetic, a protein, peptide, lipid, or surfactant, and combinations thereof. In certain embodiments, the imaging agent further comprises a detectable label.

In certain embodiments, a particle of the invention may further comprise a targeting agent or molecule. A particle may also further or alternatively comprise an adjuvant.

In certain embodiments, a particle of the invention may further comprise an agent covalently linked to the particle. The agent may be a bioactive agent, such as a drug. The agent may preferably be a hydrophilic agent, such that through its covalent linkage to the particle, the agent alters charge or hydrophilicity of the particle, e.g., to decrease the particle's mucoadhesion. The covalent linkage may be cleavable under biological conditions.

Also provided is an inhaler or nebulizer comprising a particle as described herein.

An additional aspect relates to a use of a particle as described herein in the manufacture of a medicament for the treatment, prevention, or diagnosis of a condition in a patient, including medicaments adapted for topical administration to a mucosal tissue.

An additional aspect relates to a method for transfecting a cell comprising contacting the cell with a particle of the invention that comprises a nucleic acid. A particle of the invention comprising a nucleic acid may transfect a cell at a higher efficiency, e.g., at 2, 5, 10, 20, 50, 100 or greater -fold higher efficiency, than the naked nucleic acid, e.g., in the presence of a mucosal barrier.

An additional aspect related to a method for treating, preventing, or diagnosing a condition in a patient, comprising administering to the patient a particle as described herein or a pharmaceutical composition comprising one or more such particles, e.g., by topical administration to a mucosal tissue. In certain embodiments, the particle passes through a mucosal barrier in the patient.

An exemplary method for preparing such particles may include: providing microparticles or nanoparticles comprising a pharmaceutically acceptable polymer and coupling (e.g., by coating, covalent linkage, or co-localization) to the surface of the microparticles or nanoparticles a surface-altering agent, e.g., a polyethylene glycol, a nucleic acid, a protein, or a carbohydrate. Such a method may further include: coupling (e.g., by coating, covalent linkage, or co-localization) to the particles an imaging agent, a detectable label, or a targeting moiety. The method may further include one or more of: forming a particle suspension, passing the particle suspension through a filter, removing impurities from the particle suspension, centrifugation to pellet the particles, dialyzing the particle suspension, and adjusting the pH of the particle suspension. The method may also include quenching the covalent linking reaction.

An additional aspect of the invention comprises a method of reducing the mucoadhesiveness of a substance by modifying the substance with a surface-altering moiety, such as PEG or a carbohydrate. Herein, the terms "surface-altering moiety" and "surface-altering agent" are used substantially interchangeably, wherein "surface-altering agent" referes preferentially to an individual entity and "surface-altering moiety" refers to all or part of a molecule. The surface-altering moiety may enhance the hydrophilicity of the substance. For example, in certain - further embodiments, the invention comprises identifying a therapeutic agent or particle, e.g., small molecule, nucleic acid, protein, liposome, polymer, liposome, metal, or metal oxide, the mucoadhesiveness of which is desired to be reduced. The substance may then be modified with a surface-altering agent. For example, the method may comprise identifying a moiety on the substance (e.g., small molecule, protein, liposome, polymer, liposome, or virus) to which the surface-altering agent (e.g., PEG) may be covalently attached, e.g., without losing activity, or through a bond susceptible to intracellular cleavage (e.g., hydrolytic or enzymatic), such as an ester or amide. Alternatively, the surface-altering agent may be non-covalently associated with the substance, e.g., by coating a particulate form of the substance, e.g., to promote its diffusivity through mucus. In certain further embodiments, a pharmaceutical preparation of the modified substance, e.g., a formulation adapted for topical delivery to a mucosal tissue of a patient may be administered to a patient.

An additional aspect of the invention comprises a method of increasing the diffusivity in mucus of a substance in need thereof, by modifying the substance with a surface-altering agent. For example, in certain embodiments the invention comprises selecting a substance in need of increased diffusivity through mucus, an appropriate surface-altering agent to promote diffusion of the substance through mucus, and a moiety on said substance to which the surface-altering agent may be coupled in order to increase the substance's diffusivity through mucus while avoiding the total loss of activity of the substance. The surface-altering agent may then be disposed on said substance, in order to increase its diffusivity through mucus. In addition, the substance with said surface-altering agent may be formulated to produce a pharmeceutical preparation, which may be delivered to a patient with the purpose of increasing diffusivity in mucus, e.g., in a formulation adapted for topical delivery to a mucosal tissue of a patient. Said pharmaceutical preparation or the substance with said surface-altering agent may be delivered to a mucosal surface in a patient, may pass through a mucosal barrier in the patient, and/or may exhibit prolonged residence time on a mucus-coated tissue, e.g., due to reduced mucoadhesion.

Substances in need of increased diffusivity may, for example, be hydrophobic, have many hydrogen bond donors or acceptors, or be highly charged. Such a substance may be an agent that travels through human mucus at less than or equal to one-tenth (or even one-hundredth or one-thousandth) the rate it travels through water. A number of drugs that are mucoadhesive are known in the art (Khanvilkar K, Donovan MD, Flanagan DR, Drug transfer through mucus, Advanced Drug Delivery Reviews 48 (2001) 173-193; Bhat PG, Flanagan DR, Donovan MD. Drug diffusion through cystic fibrotic mucus: steady-state permeation, rheologic properties, and glycoprotein morphology, J Pharm Sci, 1996 Jun;85(6):624-30.). As an example, dexamethasone, a corticosteriod for treating inflammation, is suggested to not be efficient because of inadequate penetration of the mucus barrier (Kennedy, M.J., Pharmacotherapy, 2001. 21(5): p. 593-603). In addition, mucus slows the diffusion of some proteins; see, for example Saltzman WM, Radomsky ML, Whaley KJ, Cone RA, Antibody Diffusion in Human Cervical Mucus, Biophysical Journal, 1994. 66:508-515.

In certain embodiments, substances (such as particles) modified with surface-altering agents as described herein may pass through a mucosal barrier in the patient, and/or exhibit prolonged residence time on a mucus-covered tissue, e.g., such substances are cleared more slowly (e.g., at least 2 times, 5 times, 10 times, or even at least 20 times more slowly) from a patient's body than a typical comparable carboxyl-modified polystyrene particle.

The present invention also contemplates the use of "sacrificial" particles or polymers to promote transport of active particles through mucus, wherein sacrificial particles or polymers increase the rate at which the active particles move through the mucus. Without wishing to be bound by theory, it is believed that such sacrificial particles interact with the mucus and alter either the structural or adhesive properties of the surrounding mucus such that the active particles experience decreased mucoadhesion. For example, the invention contemplates the use of PEG (e.g., not physically or chemically associated with the active particle(s)) as a sacrificial polymer to promote the diffusion of certain particles through mucus. In addition, the invention contemplates the use of particles lacking a surface-altering agent (and optionally lacking a therapeutic agent), used in combination with surface-altering particles of the invention, e.g., containing a therapeutic agent. In certain embodiments, sacrificial particles are carboxyl-modified polystyrene (PS) particles. In certain embodiments, the invention contemplates use of sacrificial particles which are less than 1,000,000, 500,000, 200,000, 100,000, 50,000, 20,000, 10,000, 5000, 2000, 1000, 500, 200, 100, 50, 20, 10, 5, 2, or 1 nm in diameter, or have a diameter intermediate between any of these values. In certain embodiments, the invention contemplates use of sacrificial particles that pass through a mucosal barrier at a rate that is less than 1/100 1/200, 1/500, 1/600, 1/1000, 1/2000, 1/3000, 1/5000, or even less than 1/10,000 of the rate of the particle in water under identical conditions. Further, the present invention provides sacrificial particles that travel at certain absolute rates. For example, the sacrificial particles may travel at rates less than 2, 1, 5x10⁻¹, 2x10⁻¹ , 1x10⁻¹, 8 x10⁻² , 6 x10⁻², 5x10⁻² 4 x10⁻², 2x10⁻², 1x10⁻², 5x10⁻³, 2x10⁻³, 1x10⁻³, 5x10⁻⁴, 2x10⁻⁴, 1 x 10⁻⁴, 5 x 10⁻⁵, 2 x 10⁻⁵, or even less than 1x10⁻⁵ µm²/s, at a time scale of 1s.

The present invention also contemplates a composition of matter which comprises human mucus (e.g., cervicovaginal, pulmonary, gastrointestinal, nasal, respiratory, or rectal mucus) and any of the particles described above.

The present invention also contemplates a particle comprising a polymer that includes regions of a surface-altering agent that localize to the surface of the particle. For example, a particle may be a copolymer of a mucoresistant polymer, such as PEG. Such a polymer may form a particle wherein regions that promote diffusion through mucus, are localized on the surface of the particle, thus reducing or even obviating the need for a separate coating or other modification with a surface-altering agent.

In certain embodiments, a particle may include an agent that promotes diffusion through mucus, wherein said agent is present both on the surface and inside the particle. Said agent may be attached covalently or noncovalently to another component of the particle such as a bioactive agent or a polymeric vehicle.

The invention further provides a composition comprising a first plurality of particles and a second plurality of particles. In certain embodiments, the first plurality of particles and the second plurality of particles are distinct types of particles. In certain embodiments, the first plurality of particles comprises mucoresistant particles as described above and the second plurality comprises sacrificial particles. In certain embodiments, the first plurality of particles make up at least 1%, 2%, 5%, 10%, 15%, 20%, 30%, 40%, 50%, 70%, 90%, 95%, or 99% of the total particles in the composition. In certain embodiments, the second plurality of particles make up at least 1%, 2%, 5%, 10%, 15%, 20%, 30%, 40%, 50%, 70%, 90%, 95%, or 99% of the total particles in the composition. In certain embodiments, the particles of the first plurality have one or more of the characteristics described in the preceding paragraphs.

Particles within a plurality of particles may be classified as having one of three modes of transport: diffusive, immobile, and hindered.

In certain embodiments, the second plurality of particles comprises an immobile fraction defined as those those that display an average MSD smaller than the 10-nm resolution at a time scale of 1 s. In certain embodiments, the immobile fraction may comprise greater than 80%, 70%, 60%, 50%, 40%, 30%, 20%, 10%, 5%, 2%, or 1% of the particles in the second plurality.

In certain embodiments, the second plurality of particles comprises a hindered fraction which strongly adheres to mucus but is not immobile. The sum of the hindered and immobile fractions is defined herein in Section 1.5 of the Exemplification as particles, that display RC values below the 97.5% range for either short or long time scales. In certain embodiments, the hindered fraction may comprise greater than 85%, 60%, 50%, 40%, 30%, 20%, 10%, 5%, 2%, or 1% of the particles in the second plurality. The second plurality of particles may diffuse through human cervicovaginal mucus at an average diffusivity that is less than 1/100, 1/200, 1/500, 1/1000, 1/2000, 1/5000, or 1/10000 the diffusivity that the particles diffuse through water at a time scale of 1s.

In certain embodiments, the first plurality of particles comprises a diffusive fraction which adheres weakly to mucus or does not adhere at all. The diffusive fraction is defined herein in Section 1.5 of the Exemplification as particles that are not hindered or immobile. In certain embodiments, the particles of the diffusive fraction have one or more of the mucus-resistant qualities discussed above. In certain embodiments, the diffusive fraction may comprise greater than 85%, 60%, 50%, 40%, 30%, 20%, 10%, 5%, 2%, or 1% of the particles in the first plurality.

Another aspect of the invention provides an envelope virus having a surface-altering moiety disposed on a surface of the virus (e.g., coating the surface of the virus), wherein said virus diffuses through human cervicovaginal mucus at a diffusivity (at a time scale of 1 s) that is more than5, 10, 20, 50, 100, 200, 500, or 1000-fold greater than the diffusivity at which a corresponding virus lacking the surface-altering moiety diffuses through human cervicovaginal mucus. The virus may further comprise a vector or other therapeutic nucleic acid as contemplated herein.

### Brief Description of the Drawings

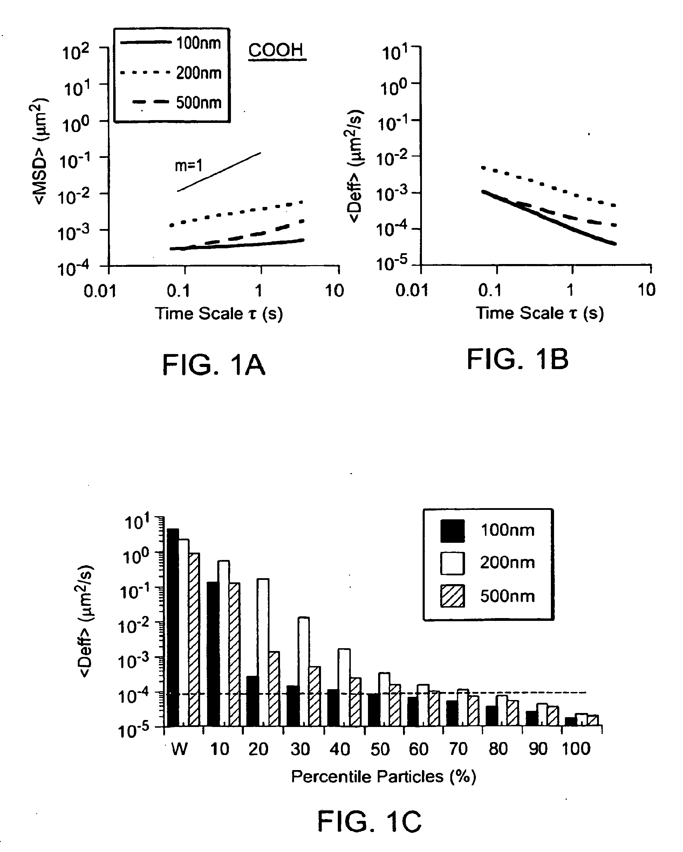

Fig. 1A, 1B, and 1C. Transport rates of COOH-modified polystyrene (COOH-PS) particles in CV mucus. (A) Ensemble-averaged geometric mean square displacements (<MSD>) and (B) effective diffusivities (<D_{eff}>) as a function of time scale. (C) Average D_{eff} of sub-fractions of particles, from fastest to slowest, at a time scale of 1s. "W" indicates the D_{eff} in pure water. The dashed black line at <D_{eff}> = 1x10⁻⁴ signifies the microscope's resolution-particles slower than this value are considered immobile. Data represent average of 3 experiments, with n ≥ 120 particles for each experiment.

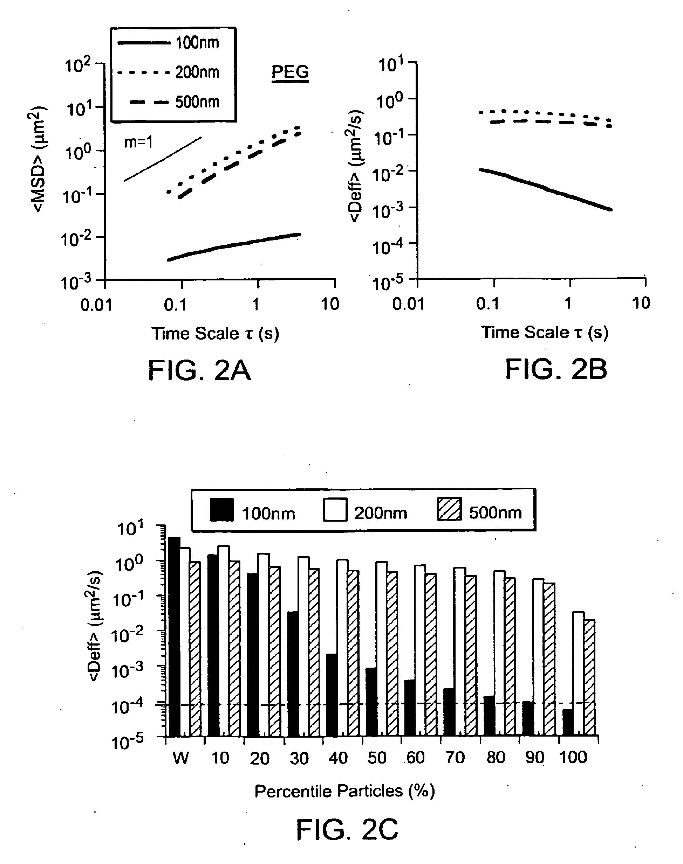

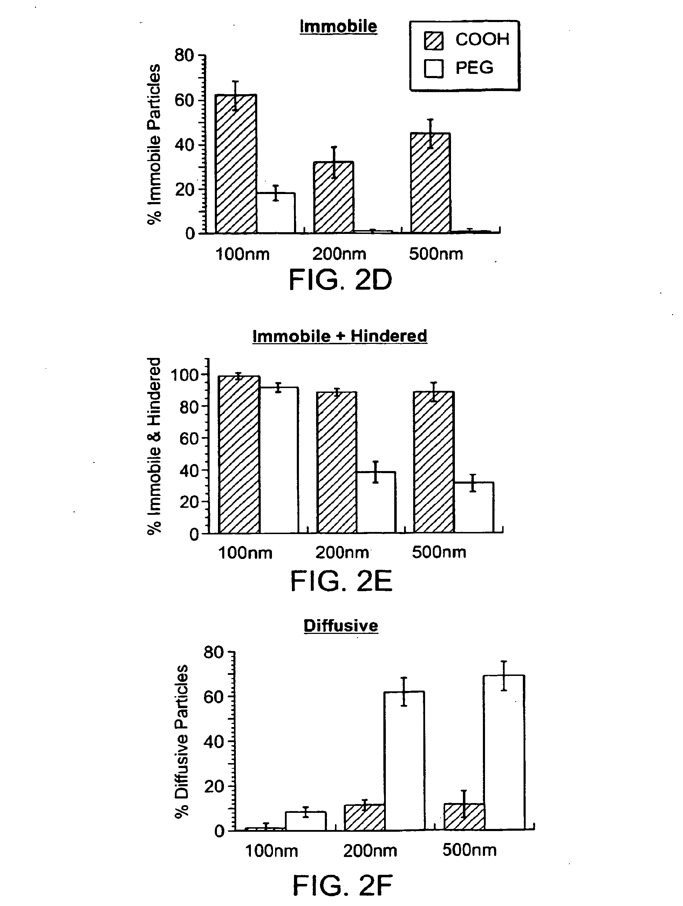

Fig. 2A, 2B, 2C, 2D, 2E, and 2F. Transport rates of polystyrene particles modified with 2kDa PEG (PEG2kDa-PS) in CV mucus. (A) Ensemble-averaged geometric mean square displacements (<MSD>) and (B) effective diffusivities (<D_{eff}>) as a function of time scale. (C) Average D_{eff} of sub-fractions of PEG2kDa-PS, from fastest to slowest, at a time scale of 1s. The dashed black line at <D_{eff}> = 1x10-4 signifies the microscope's resolution - particles slower than this value are considered immobile. Transport mode distributions of COOH-PS and PEG2kDa-PS: (D) immobile particles, (E) immobile and hindered particles, and (F) diffusive particles.

Data represent ensemble average of three experiments, with n ≥ 120 particles for each experiment.

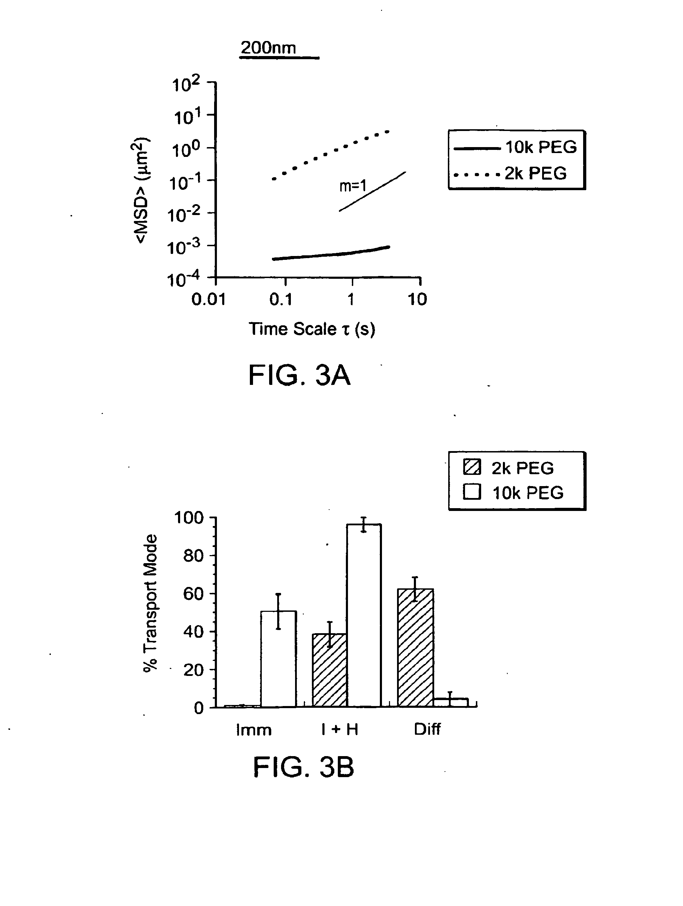

Fig. 3A and 3B. Transport rates of polystyrene particles modified with 10kDa PEG (PEG10kDa-PS) in CV mucus. (A) Ensemble-averaged geometric mean square displacements (<MSD>) as a function of time scale. (B) Fractions of PEG10kDa-PS undergoing different transport modes: immobile (Imm), immobile and hindered (I+H), and diffusive (Diff) particles. Data represent ensemble average of three experiments, with n ≥110 particles for each experiment.

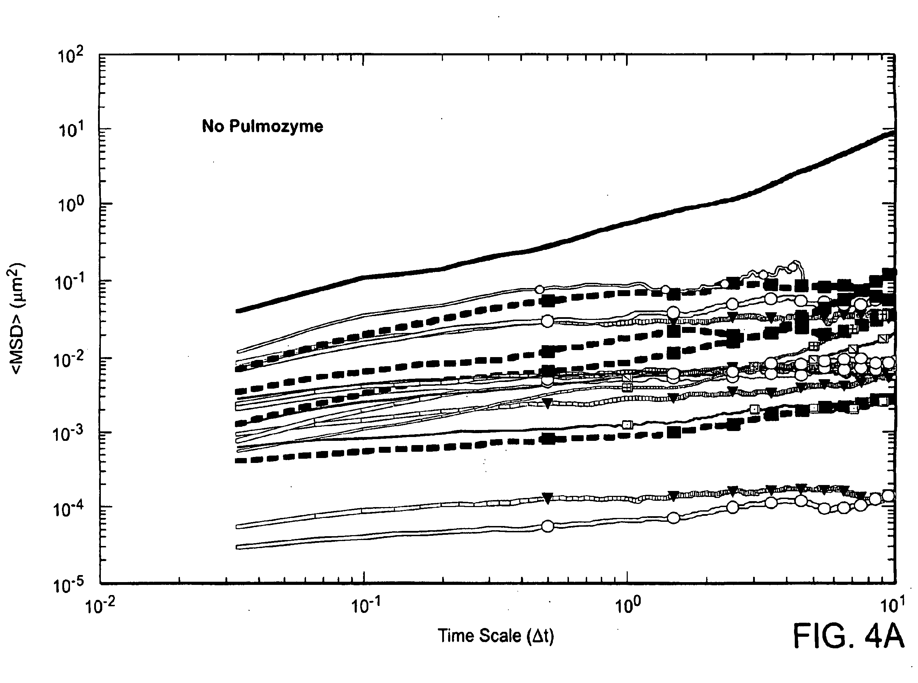

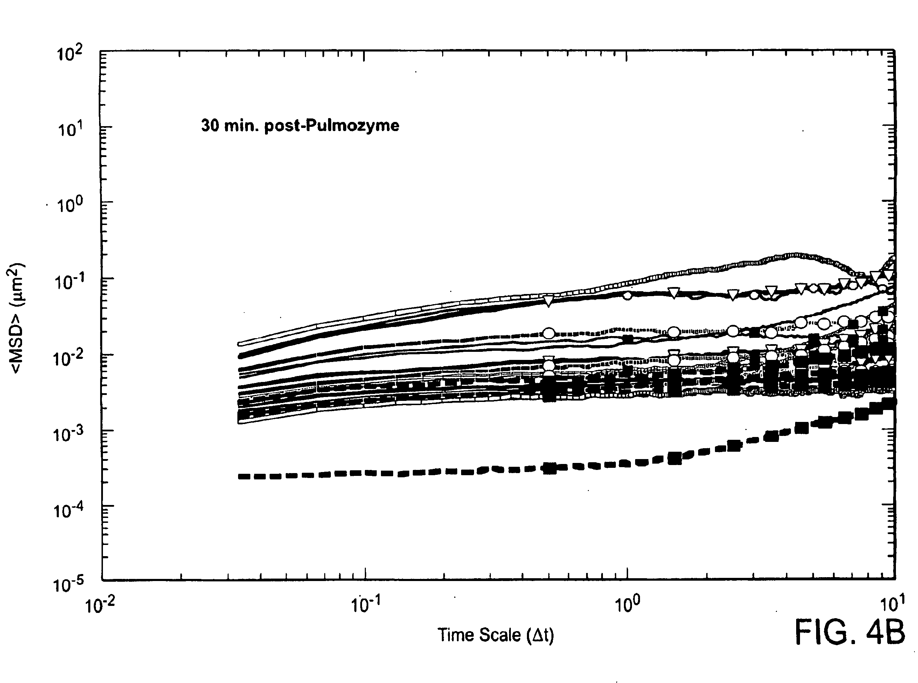

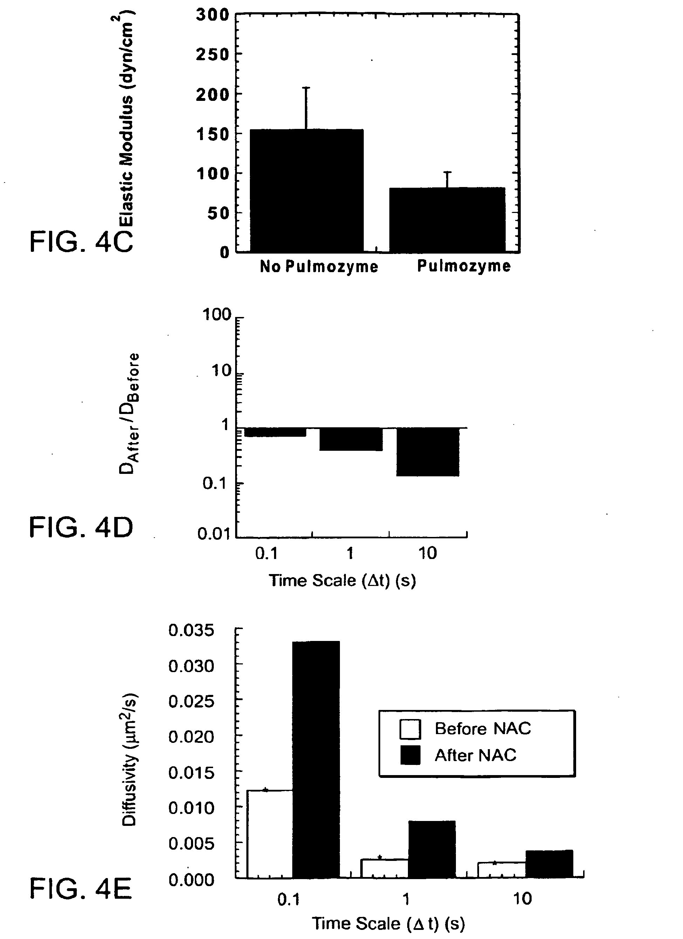

Figure 4A, 4B, 4C, 4D and 4E. Effect of mucolytics (rhDNase, NAC) on mucus rheology and particle transport in CF mucus. MSDs of a subset of individual 200nm particles for (A) no treatment (notice large variation) and (B) pulmozyme (rhDNAse) treatment (notice more uniform) (n ≥ 120). (C) Bulk viscosity was reduced ∼50% by treatment with rhDNase, but surprisingly did not correlate to improved particle transport in CF mucus (D) (see our paper in JBC for explanation [19]). Particle transport in CF mucus was dramatically improved, however, with NAC: (E) Effective diffusivities of 100nm particles (n=100-180) was increased significantly (p<0.05) at 30mins (0.4mM NAC).

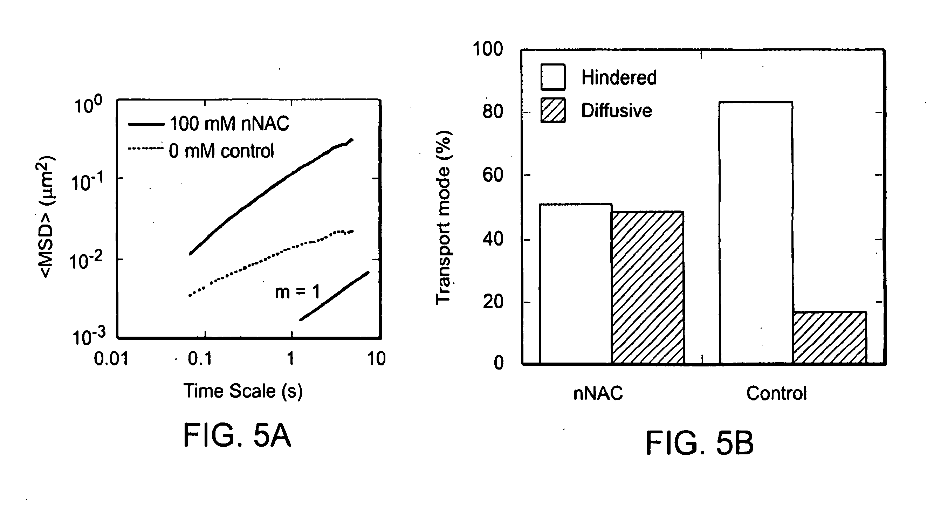

Figure 5A and 5B. Ensemble averaged transport rates of PEG-modified 500 nm polystyrene (PEG-PS) nanoparticles in undiluted lung mucus expectorated from cystic fibrosis (CF) patients. (A) Ensemble geometric mean square displacements show that pretreatment of mucus with neutralized N-acetyl-L-cysteine increased transport rates 10.7-fold compared to no treatment control (PBS). (B) Classifying the trajectories of particle motion into different transport modes (immobile, hindered, diffusive) show that the diffusive fraction of 500 nm PEG-PS is enhanced 3-fold compared to the no treatment control. For both conditions, the number of immobile particles is <3%. Data represent n = 200-250 particles per condition.

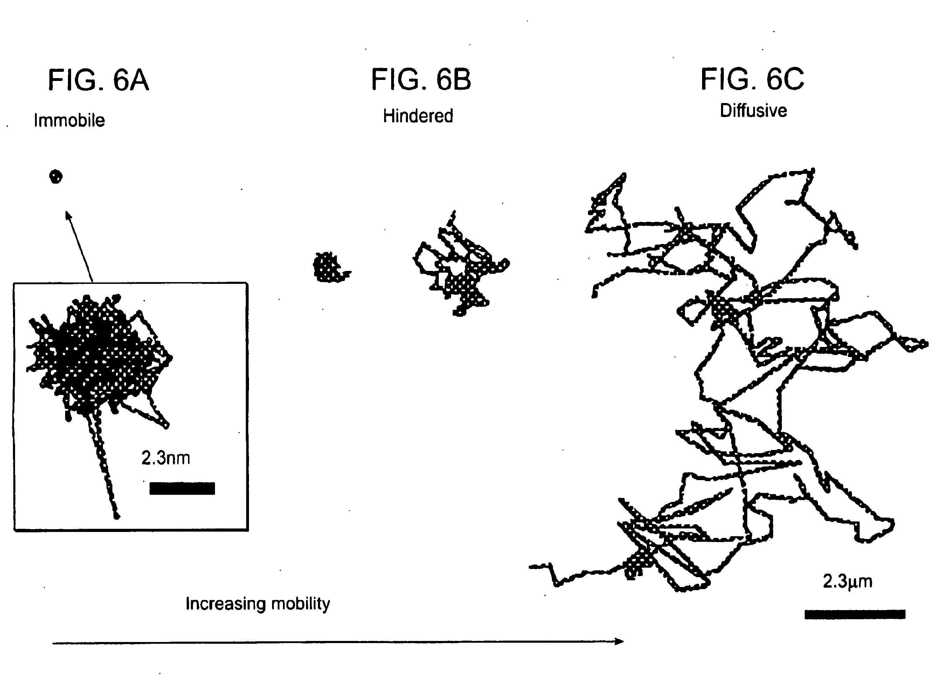

Fig. 6A, 6B, and 6C. Typical trajectories of particles undergoing transport in CV mucus: (A) immobile, (B) hindered, and (C) diffusive parcles. Scale bar represents 2.3 µm for all trajectories. Inset shows motions of immobile paricle zoomed in 1000x; scale bar in Inset represents 2.3 nm.

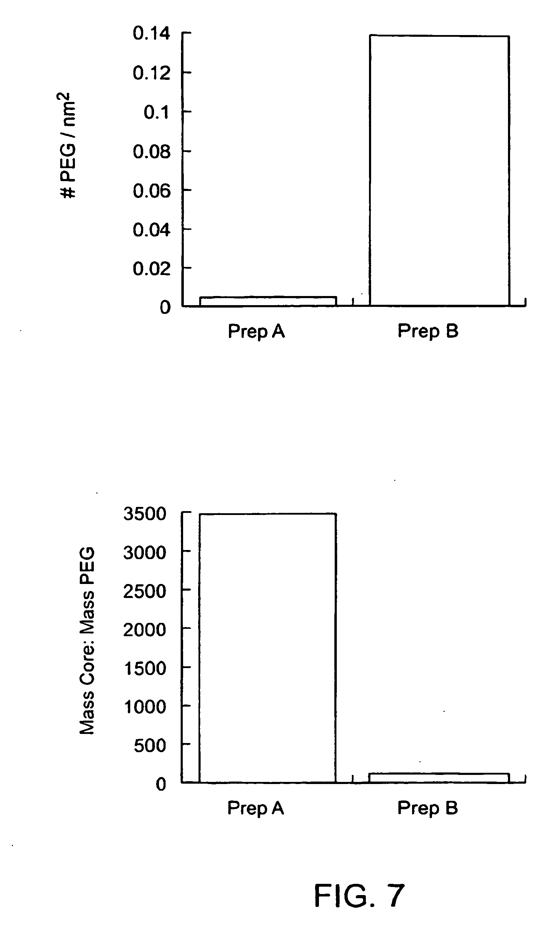

Fig. 7A and 7B. (A) Surface density of polyethylene glycol (PEG; M.W. ∼3.4 kDa) on two different particle preparations. Prep A: PEG adsorbed on to 500 nm polystyrene particles as disclosed in Example 6B in WO 2005/072710 A2. Prep B: High density PEG conjugated to 500 nm polystyrene particles as described in Lai et al, PNAS v104(5): 1482-1487. (B) Mass ratio of core polymer to surface PEG for Prep A and Prep B.

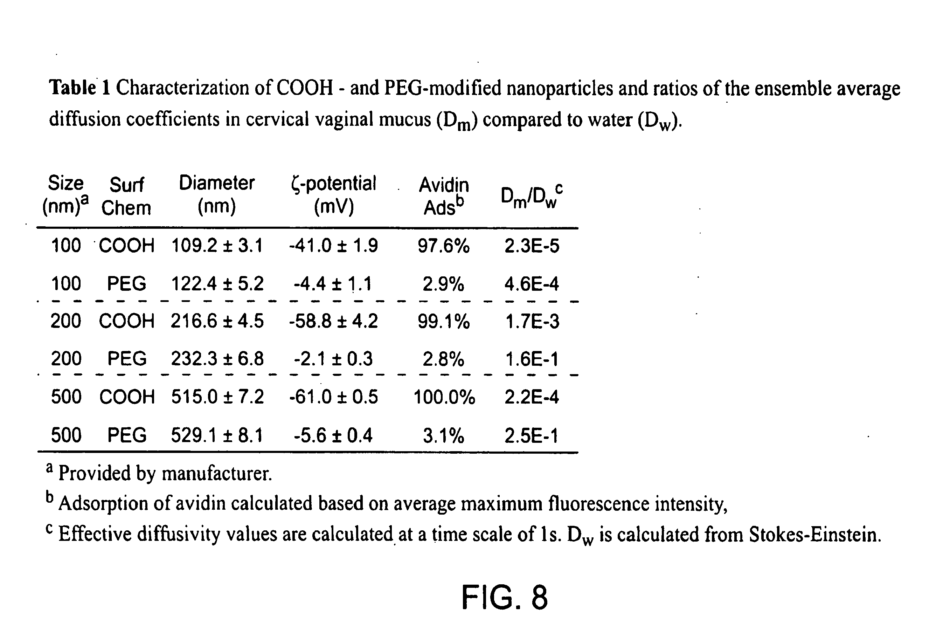

Fig. 8. Table depicting size of particles (column 1), surface chemistry of particles (COOH = uncoated, PEG = coated) (column 2), experimentally determined diameter of particles (column 3), zeta-potential of particles (column 4), avidin adsorbance of particles (column 5), and effective diffusivity at a time scale of 1 s (column 5).

### Detailed Description of the Invention

### 1. Overview

The present invention relates in part to a nanoparticle or microparticle coated with a surface agent that facilitates passage of the particle through mucus. Said nanoparticles and microparticles have a higher concentration of surface agent than has been previously achieved, leading to the unexpected property of extremely fast diffusion through mucus. The present invention further comprises a method of producing said particles. The present invention further comprises methods of using said particles to treat a patient.

Cervicovaginal (CV) mucus typically exhibits macroscopic viscosity within the range (albeit in the higher end) of typical human mucus secretions, including lungs, GI tract, nose, eyes and epididymus. This is partly attributed to the similarity in the chemical composition of various human mucuses. For example, the mucin glycoform MUC5B is the major secreted form of mucin in the mucosal layers protecting the CV tract, lungs, nose, and eye. The mucin content, approximately 1-3% by weight, is also similar between cervical, nasal and lung mucus. The composition of water in the aforementioned mucus types all falls within the range of 90-98%. The similar mucus composition and mucin glycoforms lead to similar rheology, characterized here by log-linear shear-thinning of viscosity.

Nanoparticles larger than the reported average mesh pore size of human mucus (approximately 100 nm) have been thought to be much too large to undergo rapid diffusional transport through mucus barriers. However, large nanoparticles are preferred for higher drug encapsulation efficiency and the ability to provide sustained delivery of a wider array of drugs. We disclose herein a new composition of matter comprising large nanoparticles, 500 and 200 nm in diameter, coated with a surface-modifying agent, such as polyethylene glycol. Such nanoparticles diffuse through mucus with an effective diffusion coefficient (D_{eff}) nearly as high as that for the same particles in water (at timescale τ=1s). In contrast, for uncoated particles 100-500 nm in diameter, D_{eff} was 2400- to 40,000-fold lower in mucus than in water. Thus, in contrast to the prevailing belief, these results demonstrate that large nanoparticles, if properly coated, can rapidly penetrate physiological human mucus, and offer the prospect that large nanoparticles can be used for mucosal drug delivery.

Treatments for cervicovaginal (CV) tract diseases, often based on drugs delivered to the systemic circulation via pills or injections, typically suffer from low efficacy. For example, systemic chemotherapy is typically the last or strictly concurrent option, after surgery and radiotherapy, for treatment of cervical cancer. In addition, systemic medications can lead to significant adverse side effects, when high drug concentrations in the circulation are required to elicit a therapeutic response in the CV tract. To reduce side effects and achieve localized therapy, recent efforts have increasingly emphasized topical drug delivery methods, such as creams, hydrogels, and inserted devices, to deliver therapeutics via the apical side of the cervix epithelium. Apical drug delivery may also be extended to protection against sexual transmission of infections, since neutralizing antibodies and microbicides must act at mucosal surfaces in order to block the entry of pathogens.

Nanoparticle systems possess desirable features for treatment, including: (i) sustained and controlled release of drugs locally, (ii) potential to cross the mucosal barrier due to the nanometric size, (iii) rapid intracellular trafficking to the perinuclear region of underlying cells, and (iv) protection of cargo therapeutics from degradation and removal in the mucus. However, therapeutic and/or diagnostic particles must overcome the mucosal barrier lining the cervicovaginal tract in order to reach underlying cells and avoid clearance. Mucins, highly glycosylated large proteins (10-40 MDa) secreted by epithelial cells, represent the principle component of the entangled viscoelastic gel that protects the underlying epithelia from entry of pathogens and toxins. Other mucus constituents, such as lipids, salts, macromolecules, cellular debris and water, work together with mucins to form a nanoscopically heterogeneous environment for nanoparticle transport, where the shear-dependent bulk viscosity is typically 100-10,000 times more viscous than water. Small viruses up to 55 nm have been shown to diffuse in CV mucus as rapidly as in water; however, a larger virus, 180 nm herpes simplex virus, was slowed 100- to 1000-fold by CV mucus compared to water, suggesting that the mucus mesh spacing is about 20-200 nm. It was also previously reported that polystyrene particles (59-1000 nm) adhered tightly to cervical mucus, rendering them completely immobile (Olmsted, SS, Padgett, JL, Yudin, Al, Whaley, KJ, Moench, TR & Cone, RA (2001) Biophysical Journal 81, 1930-1937). These observations have suggested that the transport of synthetic polymer nanoparticles, especially those larger than ∼59 nm, was unlikely to occur efficiently enough to allow access of sustained release particles to underlying epithelium in human mucus-covered tissues.

To investigate and potentially improve the transport of nanoparticles across the cervicovaginal mucus barrier, we studied the quantitative transport rates of hundreds of individual nanoparticles of various sizes and surface chemistries in human cervicovaginal secretions. Undiluted mucus at physiologically relevant conditions was obtained by a novel procedure that uses a menstrual collection device (Boskey, ER, Moench, TR, Hees, PS & Cone, RA (2003) Sexually Transmitted Diseases 30, 107-109). Surprisingly, we report that nanoparticles, including those larger than the previously reported CV mucus mesh spacing, are capable of rapid transport in CV mucus if they are coated with a muco-resistant polymer, such as polyethylene glycol.

High MW poly(ethylene glycol) (PEG) has been used as a mucoadhesive added to polymeric systems for its reported ability to interpenetrate into the mucus network (Bures et al., J. Controlled Release, (2001) 72:25-33; Huang et al., J. Controlled Release, (2000) 65:63-71; Peppas et al., J. Controlled Release, (1999) 62:81-87) and hydrogen bond to mucins Willits et al., Biomaterials, (2001) 22:445-452; Sanders et al., J. Controlled Release, (2003) 87:117-129, and PCT Patent Application No. US2005/002556). However, as shown in the examples below, modifying the surface of different particle types having a dense PEG coating decreased the adsorption of mucus components to the particle surface and allowed more rapid transport through mucus with a reduced number of adhesive particles. High MW poly(ethylene glycol) may be employed to reduce mucoadhesion in certain configurations, e.g., wherein the length of PEG chains extending from the surface is controlled (such that long, unbranched chains that interpenetrate into the mucus network are reduced or eliminated). For example, linear high MW PEG may be employed in the preparation of particles such that only portions of the linear strands extend from the surface of the particles (e.g., portions equivalent in length to lower MW PEG molecules). Alternatively, branched high MW PEG may be employed. In such embodiments, although the molecular weight of a PEG molecule may be high, the linear length of any individual strand of the molecule that extends from the surface of a particle would correspond to a linear chain of a lower MW PEG molecule.

PEG can be produced in a range of molecular weights. The present invention contemplates the use of one or more different molecular weights of PEG on the surface of nanoparticles, including but not limited to 300 Da, 600 Da, 1 kDa, 2 kDa, 3 kDa, 4 kDa, 6 kDa, 8 kDa, 10 kDa, 15 kDa, 20 kDa, 30 kDa, 50 kDa, 100 kDa, 200 kDa, 500 kDa, and 1 MDa. In addition, PEG of any given molecular weight may vary in other characteristics such as length, density, and branching. This invention contemplates the use of different variants of PEG, including PEG of different lengths, densities, or branchedness.

While not wishing to be bound by theory, one possible mechanism for this effect is that PEG alters the microenvironment of the particle, for example by ordering water and other molecules in the particle/mucus environment; an additional or alternative possible mechanism is that free PEG shields the adhesive domains of the mucin fibers, thereby reducing particle adhesion and speeding up particle transport.

Modification of particle surface with other polymers, proteins, surfactants, sugars, carbohydrates, nucleic acids, or non-mucoadhesive materials may also result in increased transport in mucus and other adhesive biological fluids, such as serum. In certain embodiments, the particle surface is coated with one or more of DNA, RNA, bovine serum albumin (BSA), human serum albumin (HSA), poly-glycine, polyglycolic acid, agar, agarose, alginic acid, amylopectin, amylose, beta-glucan, callose, carrageenan, cellodextrins, cellulin, cellulose, chitin, chitosan, chrysolaminarin, curdlan, cyclodextrin, dextrin, ficoll, fructan, fucoidan, galactomannan, gellan gum, glucan, glucomannan, glycocalyx, glycogen, hemicellulose, hydroxyethyl starch, kefiran, laminarin, mucilage, glycosaminoglycan, natural gum, paramylon, pectin, polysaccharide peptide, schizophyllan, sialyl lewis x, starch, starch gelatinization, sugammadex, xanthan gum, and xyloglucan. For example, as shown below, modification of particle surface by the covalent attachment of PEG to COOH-modified particles increases transport in mucus. Furthermore, addition of N-Acetyl Cysteine increases transport in mucus. Other molecules such as surfactants or polymers, including poly(aspartic acid), and proteins, such as heparin, may also increase transport rates in mucus.

Accordingly, the present invention relates to particles (for example, polymeric or liposomal particles) and compositions comprising them, such as pharmaceutical compositions for the delivery of biologically active and/or therapeutic agents, e.g., for the prevention, detection or treatment of a disease or other condition in a patient, particularly, for delivery across mucosal barriers in the patient. The present invention also provides a particle comprising a polymer having regions of polyethylene glycol that are presented on the surface of the particle. In certain embodiments, biodegradable and/or biocompatible polymers may be used to transport or carry an adsorbed or encapsulated therapeutic agent across a mucosal barrier present in any mucosal surface, e.g., gastrointestinal, nasal, respiratory, rectal, or vaginal mucosal tissues in a patient. Agents that may be adsorbed or encapsulated in the subject compositions include imaging and diagnostic agents (such as radioopaque agents, labeled antibodies, labeled nucleic acid probes, dyes, such as colored or fluorescent dyes, etc.) and adjuvants (radiosensitizers, transfection-enhancing agents, chemotactic agents and chemoattractants, peptides that modulate cell adhesion and/or cell mobility, cell permeabilizing agents, vaccine potentiators, inhibitors of multidrug -resistance and/or efflux pumps, etc.). The present invention also relates to methods of making and/or administering such compositions, e.g., as part of a treatment regimen, for example, by inhalation, topically (e.g., for adminstration to a mucosal tissue of a patient), or by injection, e.g., subcutaneously, intramuscularly, or intravenously.

### 2. Definitions

For convenience, before further description of the present invention, certain terms employed in the specification, examples, and appended claims are collected here. These definitions should be read in light of the remainder of the disclosure and understood as by a person of skill in the art.

The term "access device" is an art-recognized term and includes any medical device adapted for gaining or maintaining access to an anatomic area. Such devices are familiar to artisans in the medical and surgical fields. An access device may be a needle, a catheter, a cannula, a trocar, a tubing, a shunt, a drain, or an endoscope such as an otoscope, nasopharyngoscope, bronchoscope, or any other endoscope adapted for use in the head and neck area, or any other medical device suitable for entering or remaining positioned within the preselected anatomic area.

The terms "biocompatible polymer" and "biocompatibility" when used in relation to polymers are art-recognized. For example, biocompatible polymers include polymers that are neither themselves toxic to the host (e.g., an animal or human), nor degrade (if the polymer degrades) at a rate that produces monomeric or oligomeric subunits or other byproducts at toxic concentrations in the host. In certain embodiments of the present invention, biodegradation generally involves degradation of the polymer in an organism, e.g., into its monomeric subunits, which may be known to be effectively non-toxic. Intermediate oligomeric products resulting from such degradation may have different toxicological properties, however, or biodegradation may involve oxidation or other biochemical reactions that generate molecules other than monomeric subunits of the polymer. Consequently, in certain embodiments, toxicology of a biodegradable polymer intended for in vivo use, such as implantation or injection into a patient, may be determined after one or more toxicity analyses. It is not necessary that any subject composition have a purity of 100% to be deemed biocompatible. Hence, a subject composition may comprise 99%, 98%, 97%, 96%, 95%, 90% 85%, 80%, 75% or even less of biocompatible polymers, e.g., including polymers and other materials and excipients described herein, and still be biocompatible.

To determine whether a polymer or other material is biocompatible, it may be necessary to conduct a toxicity analysis. Such assays are well known in the art. One example of such an assay may be performed with live carcinoma cells, such as GT3TKB tumor cells, in the following manner: the sample is degraded in 1M NaOH at 37 °C until complete degradation is observed. The solution is then neutralized with 1 M HCl. About 200 µL of various concentrations of the degraded sample products are placed in 96-well tissue culture plates and seeded with human gastric carcinoma cells (GT3TKB) at 10⁴/well density. The degraded sample products are incubated with the GT3TKB cells for 48 hours. The results of the assay may be plotted as % relative growth vs. concentration of degraded sample in the tissue-culture well. In addition, polymers and formulations of the present invention may also be evaluated by well-known in vivo tests, such as subcutaneous implantations in rats to confirm that they do not cause significant levels of irritation or inflammation at the subcutaneous implantation sites.

Exemplary biocompatible and biodegradable polymers disclosed in U.S. Patent 7,163,697 may be employed to make the polymeric particles of the present invention.

The term "biodegradable" is art-recognized, and includes polymers, compositions and formulations, such as those described herein, that are intended to degrade during use. Biodegradable polymers typically differ from non-biodegradable polymers in that the former may degrade during use. In certain embodiments, such use involves in vivo use, such as in vivo therapy, and in other certain embodiments, such use involves in vitro use. In general, degradation attributable to biodegradability involves the degradation of a biodegradable polymer into its component subunits, or digestion, e.g., by a biochemical process, of the polymer into smaller, non-polymeric subunits. In certain embodiments, two different types of biodegradation may generally be identified. For example, one type of biodegradation may involve cleavage of bonds (whether covalent or otherwise) in the polymer backbone. In such biodegradation, monomers and oligomers typically result, and even more typically, such biodegradation occurs by cleavage of a bond connecting one or more of subunits of a polymer. In contrast, another type of biodegradation may involve cleavage of a bond (whether covalent or otherwise) internal to sidechain or that connects a side chain to the polymer backbone. For example, a therapeutic agent or other chemical moiety attached as a side chain to the polymer backbone may be released by biodegradation. In certain embodiments, one or the other or both general types of biodegradation may occur during use of a polymer.

As used herein, the term "biodegradation" encompasses both general types of biodegradation. The degradation rate of a biodegradable polymer often depends in part on a variety of factors, including the chemical identity of the linkage responsible for any degradation, the molecular weight, crystallinity, biostability, and degree of cross-linking of such polymer, the physical characteristics (e.g., shape and size) of the implant, and the mode and location of administration. For example, the greater the molecular weight, the higher the degree of crystallinity, and/or the greater the biostability, the biodegradation of any biodegradable polymer is usually slower. The term "biodegradable" is intended to cover materials and processes also termed "bioerodible."

In certain embodiments wherein the biodegradable polymer also has a therapeutic agent or other material associated with it, the biodegradation rate of such polymer may be characterized by a release rate of such materials. In such circumstances, the biodegradation rate may depend on not only the chemical identity and physical characteristics of the polymer, but also on the identity of material(s) incorporated therein.

In certain embodiments, polymeric formulations of the present invention biodegrade within a period that is acceptable in the desired application. In certain embodiments, such as in vivo therapy, such degradation occurs in a period usually less than about five years, one year, six months, three months, one month, fifteen days, five days, three days, or even one day or less (e.g., 4-8 hours) on exposure to a physiological solution with a pH between 6 and 8 having a temperature of between 25 and 37°C. In other embodiments, the polymer degrades in a period of between about one hour and several weeks, depending on the desired application.

The term "cervicovaginal mucus" is art-recognized and refers to fresh, minimally diluted non-ovulatory cervicovaginal mucus collected from a human subject.

The term "corresponding particle" is used herein to refer to a particle that is substantially identical to a particle to which it is compared, but typically lacking a mucoresistant surface modification. A corresponding particle may be of similar material, density, and size as the particle to which it is compared. In certain embodiments, a corresponding particle is a carboxyl-modified polystyrene (PS) particle, e.g., available from Molecular Probes, Eugene, OR. In certain embodiments, a comparable particle is a polystyrene particle that has either carboxyl, amine or sulfate aldehyde surface modifications. Said carboxyl groups are preferably present at a density of 1.77 to 6.69 carboxyls per nm² . In certain embodiments, a corresponding particle is polymeric, liposomal, viral, metal, metal oxide (e.g., silica), or a quantum dot that differs substantially only in a specified way, such as the lack of a mucoresistant surface modification.

The term "DNA" is art-recognized and refers herein to a polymer of deoxynucleotides. Examples of DNA include plasmids, gene therapy vector, and a vector designed to induce RNAi.

The term "diameter" is art-recognized and is used herein to refer to either of the physical diameter or the hydrodynamic diameter of the entity in question. The diameter of an essentially spherical particle may refer to the physical or hydrodynamic diameter. The diameter of a nonspherical particle may refer preferentially to the hydrodynamic diameter. As used herein, the diameter of a non-spherical particle may refer to the largest linear distance between two points on the surface of the particle. When referring to multiple particles, the diameter of the particles typically refers to the average diameter of the particles referred to.

The term "drug delivery device" is an art-recognized term and refers to any medical device suitable for the application of a drug or therapeutic agent to a targeted organ or anatomic region. The term includes, without limitation, those formulations of the compositions of the present invention that release the therapeutic agent into the surrounding tissues of an anatomic area. The term further includes those devices that transport or accomplish the instillation of the compositions of the present invention towards the targeted organ or anatomic area, even if the device itself is not formulated to include the composition. As an example, a needle or a catheter through which the composition is inserted into an anatomic area or into a blood vessel or other structure related to the anatomic area is understood to be a drug delivery device. As a further example, a stent or a shunt or a catheter that has the composition included in its substance or coated on its surface is understood to be a drug delivery device.

When used with respect to a therapeutic agent or other material, the term "sustained release" is art-recognized. For example, a subject composition which releases a substance over time may exhibit sustained release characteristics, in contrast to a bolus type administration in which the entire amount of the substance is made biologically available at one time. For example, in particular embodiments, upon contact with body fluids including blood, spinal fluid, mucus secretions, lymph or the like, the polymer matrices (formulated as provided herein and otherwise as known to one of skill in the art) may undergo gradual or delayed degradation (e.g., through hydrolysis) with concomitant release of any material incorporated therein, e.g., an therapeutic and/or biologically active agent, for a sustained or extended period (as compared to the release from a bolus). This release may result in prolonged delivery of therapeutically effective amounts of any incorporated therapeutic agent.

The term "delivery agent" is an art-recognized term, and includes molecules that facilitate the intracellular delivery of a therapeutic agent or other material. Examples of delivery agents include: sterols (e.g., cholesterol) and lipids (e.g., a cationic lipid, virosome or liposome).

The term "lipid" is art-recognized and is used herein to refer to a fat soluble naturally occurring moleucle. "Lipid" is also used herein to refer to a molecule with a charged portion and a hydrophobic hydrocarbon chain. Herein, the term "lipid" includes the molecules comprising liposomes.

The term "metal" is art-recognized and is used herein to refer to generally to elements in Groups 1-13/Groups I-IIIA and I-VIIIB (including transition metals, lanthanides, actinides, alkali metals, and alkaline earth metals), as well as silicon, germanium, tin, lead, antimony, bismuth, and polonium. Herein, iron, copper, silver, platinum, vanadium, ruthenium, manganese, barium, boron, lanthanides, rhenium, technetium, silicon, and others are considered metals. The term "metal oxides" as used herein refers to oxides of such metals, including silica (silicon dioxide), alumina (aluminum oxide), barium oxide, etc.

The term "microspheres" is art-recognized, and includes substantially spherical colloidal structures, e.g., formed from biocompatible polymers such as subject compositions, having a size ranging from about one or greater up to about 1000 microns. In general, "microcapsules," also an art-recognized term, may be distinguished from microspheres, because microcapsules are generally covered by a substance of some type, such as a polymeric formulation. The term "microparticles" is also art-recognized, and includes microspheres and microcapsules, as well as structures that may not be readily placed into either of the above two categories, all with dimensions on average of less than about 1000 microns. A microparticle may be spherical or nonspherical and may have any regular or irregular shape. If the structures are less than about one micron in diameter, then the corresponding art-recognized terms "nanosphere," "nanocapsule," and "nanoparticle" may be utilized. In certain embodiments, the nanospheres, nanocapsules and nanoparticles have an average diameter of about 500 nm, 200 nm, 100, 50 nm, 10 nm, or 1 nm.

A composition comprising microparticles or nanoparticles may include particles of a range of particle sizes. In certain embodiments, the particle size distribution may be uniform, e.g., within less than about a 20% standard deviation of the median volume diameter, and in other embodiments, still more uniform, e.g., within about 10% of the median volume diameter.

The term "mucolytic agent" is art-recognized, and includes substances that are used clinically to increase the rate of mucus clearance (Hanes, J., M. Dawson, Y. Har-el, J. Suh, and J. Fiegel, Gene Delivery to the Lung. Pharmaceutical Inhalation Aerosol Technology, A.J.Hickey, Editor. Marcel Dekker Inc.: New York, 2003: p. 489-539). Such substances include, for example, N-Acetyle Cysteine (NAC), which cleaves disulphide and sulfhydryl bonds present in mucin. Additional examples of mucolytics include mugwort, bromelain, papain, clerodendrum, acetylcysteine, bromhexine, carbocisteine, eprazinone, mesna, ambroxol, sobrerol, domiodol, letosteine, stepronin, tiopronin, gelsolin, thymosin β4, dornase alfa, neltenexine, erdosteine, and various DNases including rhDNase.

The term "mucus" is art-recognized and is used herein to refer to a natural substance that is viscous and comprises mucin glycoproteins. Mucus may be found in a human or a nonhuman animal, such as primates, mammals, and vertebrates. Mucus may be found in a healthy or diseased human or nonhuman animal. Mucus may be cervicovaginal, pulmonary, gastrointestinal, nasal, respiratory, or rectal. The term "mucus" as used herein refers to fresh, undiluted mucus unless otherwise specified.

The term "mucus-resistant" is used herein to refer to the property of having reduced or low mucoadhesion, or to the property of having high or increased rate of diffusion through mucus. "Mucus-resistant" may be used herein to refer to a particle that diffuses through human cervicovaginal mucus at a rate that is greater than 1/1000, 1/500, 1/20, 1/10, 1/5, or 1/2 the rate that the particle diffuses through water. "Mucus-resistant" may additionally be used herein to refer to a particle that moves in mucus at a rate more than 1 x10⁻³, 2 x10⁻³ ,5x10⁻³, 1x10⁻², 2x10⁻², 2 x10⁻², 4x10⁻², 1x10⁻¹, 2x10⁻¹ , 5x10⁻¹,1, or 2 µm²/s at a time scale of 1s. "Mucus-resistant" may additionally be used herein to refer to a particle that diffuses through a mucosal barrier at a greater rate than a corresponding non-mucus-resistant particle, e.g. a carboxyl-modified polystyrene particle of similar size and density wherein the carboxyl modifications are present at a density of 1.77 to 6.69 carboxyls per nm², wherein the mucus-resistant particle passes through a mucosal barrier at a rate that is at least 10, 20, 30, 50, 100, 200, 500, 1000, 2000, 5000, 10000-or greater fold higher than said corresponding non-mucus-resistant particle, e.g. a carboxyl-modified polystyrene particle of similar size and density wherein the carboxyl modifications are present at a density of 1.77 to 6.69 carboxyls per nm². Said corresponding non-mucus-resistant particle may also be an amine-modified polystyrene particle or a sulfate-aldehyde-modified polystyrene particle.

The term "nucleic acid" is used herein to refer to DNA or RNA including plasmids, gene therapy vectors, siRNA expression constructs, and siRNAs.

The term "nucleic acid analog" is used herein to refer to non-natural variants of nucleic acids including morpholinos, 2'O-modified nucleic acids, and peptide nucleic acids (PNAs)

The term "particle" is art-recognized, and includes, for example, polymeric particles, liposomes, metals, and quantum dots. A particle may be spherical or nonspherical. A particle may be used, for example, for diagnosing a disease or condition, treating a disease or condition, or preventing a disease or condition.

The phrases "parenteral administration" and "administered parenterally" are art-recognized terms, and include modes of administration other than enteral and topical administration, such as injections, and include without limitation intravenous, intramuscular, intrapleural, intravascular, intrapericardial, intraarterial, intrathecal, intracapsular, intraorbital, intracardiac, intradennal, intraperitoneal, transtracheal, subcutaneous, subcuticular, intra-articular, subcapsular, subarachnoid, intraspinal and intrasternal injection and infusion.

The term "peptidomimetic" is art-recognized and refers to a small protein-like chain designed to mimic a peptide. A peptidomimetic may incoporate modifications such as altered backbones and the incorporation of nonnatural amino acids.

The term "peptide" is art-recognized and refers to a polymer of amino acids. A peptide may be a protein, polypeptide, and/or oligopeptide.

The term "RNA" is art-recognized and refers herein to a ribonucleic acid. RNA may include, for example, mRNA, the transcript of an RNAi construct, or an siRNA.

The term "sacrificial agent" is used herein to refer to an agent that promotes transport of active particles through mucus, e.g., increase the rate at which the active particles move through the mucus, without degrading the mucus (e.g., is not a mucolytic agent). Without wishing to be bound by theory, it is believed that such sacrificial particles interact with the mucus and alter either the structural or adhesive properties of mucus such that the active particles experience decreased mucoadhesion. A sacrificial agent may be a particle (e.g., a microparticle or a nanoparticle) or a polymer (including, for example, PEG).

"SiRNA" is used herein to refer to an exogenous double-stranded RNA of approximately 20-25 nucleotides that decreases expression of one or more genes by base-pairing with the mRNA of said gene(s) and causing degradation of the target mRNA.

The term "surfactant" is art-recognized and herein refers to an agent that lowers the surface tension of a liquid.

The term "therapeutic agent" is art-recognized and may comprise a nucleic acid, a nucleic acid analog, a small molecule, a peptidomimetic, a protein, peptide, lipid, or surfactant, and a combination thereof.

The term "treating" is art-recognized and includes preventing a disease, disorder or condition from occurring in an animal which may be predisposed to the disease, disorder and/or condition but has not yet been diagnosed as having it; inhibiting the disease, disorder or condition, e.g., impeding its progress; and relieving the disease, disorder, or condition, e.g., causing regression of the disease, disorder and/or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain.

The term "targeting moiety" is art-recognized and is used herein to refer to a moiety that localizes to or away from a specific locale. Said moiety may be, for example, a protein, nucleic acid, nucleic acid analog, carbohydrate, or small molecule. Said entity may be, for example, a therapeutic compound such as a small molecule, or a diagnostic entity such as a detectable label. Said locale may be a tissue, a particular cell type, or a subcellular compartment. In one embodiment, the targeting moiety directs the localization of an active entity. Said active entity may be a small molecule, protein, polymer, or metal. Said active entity may be useful for therapeutic or diagnostic purposes.

Viscosity is understood herein as it is recognized in the art to be the internal friction of a fluid or the resistance to flow exhibited by a fluid material when subjected to deformation. The degree of viscosity of the polymer can be adjusted by the molecular weight of the polymer, as well as by varying the proportion of its various monomer subunits; other methods for altering the physical characteristics of a specific polymer will be evident to practitioners of ordinary skill with no more than routine experimentation. The molecular weight of the polymer used in the composition of the invention can vary widely, depending on whether a rigid solid state (higher molecular weights) is desirable, or whether a fluid state (lower molecular weights) is desired.

The phrase "pharmaceutically acceptable" is art-recognized. In certain embodiments, the term includes compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" is art-recognized, and includes, for example, pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose glucose and sucrose; (2) starches, such as com starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) cocoa butter and suppository waxes; (9) oils, such as peanut oil, cottonseed oil, sunflower oil, sesame oil, olive oil, com oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically acceptable salts" is art-recognized, and includes relatively non-toxic, inorganic and organic acid addition salts of compositions, including without limitation, analgesic agents, therapeutic agents, other materials and the like. Examples of pharmaceutically acceptable salts include those derived from mineral acids, such as hydrochloric acid and sulfuric acid, and those derived from organic acids, such as ethanesulfonic acid, benzenesulfonic acid, p-toluenesulfonic acid, and the like. Examples of suitable inorganic bases for the formation of salts include the hydroxides, carbonates, and bicarbonates of ammonia, sodium, lithium, potassium, calcium, magnesium, aluminum, zinc and the like. Salts may also be formed with suitable organic bases, including those that are non-toxic and strong enough to form such salts. For purposes of illustration, the class of such organic bases may include mono-, di-, and trialkylamines, such as methylamine, dimethylamine, and triethylamine; mono-, di- or trihydroxyalkylamines such as mono-, di-, and triethanolamine; amino acids, such as arginine and lysine; guanidine; N-methylglucosamine; N-methylglucamine; L-glutamine; N-methylpiperazine; morpholine; ethylenediamine; N-benzylphenethylamine; (trihydroxymethyl)aminoethane; and the like. See, for example, J. Pharm. Sci. 66: 1-19 (1977), incorporated herein by reference.

A "patient," "subject," or "host" to be treated by the subject method may mean either a human or non-human animal, such as primates, mammals, and vertebrates.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if it is administered after manifestation of the unwanted condition, the treatment is therapeutic, (i.e., it is intended to diminish, ameliorate, or stabilize the existing unwanted condition or side effects thereof).

The term "preventing" is art-recognized, and when used in relation to a condition, such as a local recurrence (e.g., pain), a disease such as cancer, a syndrome complex such as heart failure or any other medical condition, is well understood in the art, and includes administration of a composition which reduces the frequency of, or delays the onset of, symptoms of a medical condition in a subject relative to a subject which does not receive the composition. Thus, prevention of cancer includes, for example, reducing the number of detectable cancerous growths in a population of patients receiving a prophylactic treatment relative to an untreated control population, and/or delaying the appearance of detectable cancerous growths in a treated population versus an untreated control population, e.g., by a statistically and/or clinically significant amount. Prevention of an infection includes, for example, reducing the number of diagnoses of the infection in a treated population versus an untreated control population, and/or delaying the onset of symptoms of the infection in a treated population versus an untreated control population. Prevention of pain includes, for example, reducing the magnitude of, or alternatively delaying, pain sensations experienced by subjects in a treated population versus an untreated control population.

The phrase "prolonged residence time" is art-recognized and refers to an increase in the time required for an agent to be cleared from a patient's body, or organ or tissue of that patient. In certain embodiments, "prolonged residence time" refers to an agent that is cleared with a half-life that is 10%, 20%, 50% or 75% longer than a standard of comparison such as a comparable agent without a mucus-resistant coating. In certain embodiments, "prolonged residence time" refers to an agent that is cleared with a half-life of 2, 5, 10, 20, 50, 100, 200, 500, 1000, 2000, 5000, or 10000 times longer than a standard of comparison such as a comparable agent without a mucus-resistant coating.

The term "protein" is art-recognized and is used herein to refer to a polymer of amino acids.

The phrases "systemic administration," "administered systemically," "peripheral administration" and "administered peripherally" are art-recognized, and include the administration of a subject composition, therapeutic or other material at a site remote from the disease being treated. Administration of an agent directly into, onto, or in the vicinity of a lesion of the disease being treated, even if the agent is subsequently distributed systemically, may be termed "local" or "topical" or "regional" administration, other than directly into the central nervous system, e.g., by subcutaneous administration, such that it enters the patient's system and, thus, is subject to metabolism and other like processes.

The phrase "therapeutically effective amount" is an art-recognized term. In certain embodiments, the term refers to an amount of the therapeutic agent that, when incorporated into a polymer of the present invention, produces some desired effect at a reasonable benefit/risk ratio applicable to any medical treatment. In certain embodiments, the term refers to that amount necessary or sufficient to eliminate or reduce sensations of pain for a period of time. The effective amount may vary depending on such factors as the disease or condition being treated, the particular targeted constructs being administered, the size of the subject, or the severity of the disease or condition. One of ordinary skill in the art may empirically determine the effective amount of a particular compound without necessitating undue experimentation.

The term "ED₅₀" is art-recognized. In certain embodiments, ED₅₀ means the dose of a drug that produces 50% of its maximum response or effect, or, alternatively, the dose that produces a pre-determined response in 50% of test subjects or preparations.

The term "LD₅₀" is art-recognized. In certain embodiments, LD₅₀ means the dose of a drug that is lethal in 50% of test subjects. The term "therapeutic index" is an art-recognized term that refers to the therapeutic index of a drug, defined as LD₅₀/ED₅₀.

The terms "incorporated" and "encapsulated" are art-recognized when used in reference to a therapeutic agent, or other material and a polymeric composition, such as a composition of the present invention. In certain embodiments, these terms include incorporating, formulating, or otherwise including such agent into a composition that allows for release, such as sustained release, of such agent in the desired application. The terms contemplate any manner by which a therapeutic agent or other material is incorporated into a polymer matrix, including for example: attached to a monomer of such polymer (by covalent, ionic, or other binding interaction), physical admixture, enveloping the agent in a coating layer of polymer, and having such monomer be part of the polymerization to give a polymeric formulation, distributed throughout the polymeric matrix, appended to the surface of the polymeric matrix (by covalent or other binding interactions), encapsulated inside the polymeric matrix, etc. The term "co-incorporation" or "co-encapsulation" refers to-the incorporation of a therapeutic agent or other material and at least one other therapeutic agent or other material in a subject composition.

More specifically, the physical form in which any therapeutic agent or other material is encapsulated in polymers may vary with the particular embodiment. For example, a therapeutic agent or other material may be first encapsulated in a microsphere and then combined with the polymer in such a way that at least a portion of the microsphere structure is maintained. Alternatively, a therapeutic agent or other material may be sufficiently immiscible in the polymer of the invention that it is dispersed as small droplets, rather than being dissolved, in the polymer. Any form of encapsulation or incorporation is contemplated by the present invention, in so much as the release, preferably sustained release, of any encapsulated therapeutic agent or other material determines whether the form of encapsulation is sufficiently acceptable for any particular use.

The term "biocompatible plasticizer" is art-recognized, and includes materials which are soluble or dispersible in the compositions of the present invention, which increase the flexibility of the polymer matrix, and which, in the amounts employed, are biocompatible. Suitable plasticizers are well known in the art and include those disclosed in U.S. Pat. Nos. 2,784,127 and 4,444,933. Specific plasticizers include, by way of example, acetyl tri-n-butyl citrate (c. 20 weight percent or less), acetyltrihexyl citrate (c. 20 weight percent or less), butyl benzyl phthalate, dibutylphthalate, dioctylphthalate, n-butyryl tri-n-hexyl citrate, diethylene glycol dibenzoate (c. 20 weight percent or less) and the like.

### 3. Particles and Related Compositions

The present invention provides particles, such as microparticles or nanoparticles having one or more surface-altering moieties disposed on the outer surface wherein (a) the mass of the surface-altering moiety makes up at least 1/3400 of the mass of the particle, and/or (b) the surface-altering moiety is present on the outer surface at a density of greater than 0.01 units per nanometer squared. In certain embodiments, a polymeric particle comprises a pharmaceutically acceptable polymer, a bioactive agent, and a surface-altering agent that makes the surface of the polymeric particle mucus resistant. In alternative embodiments, a polymeric particle comprises a pharmaceutically acceptable polymer and a surface-altering agent that is also a bioactive agent. In certain such embodiments, the particle further comprises an adhesion-promoting agent, such as dimethyldioctadecyl-ammonium bromide or other cation-bearing additives, that promotes adhesion of the surface-altering agent to the surface of the particle. The surface-altering agent may increase particle transport rates in mucus.

Examples of the surface-altering agents include but are not limited to anionic protein (e.g., bovine serum albumin), surfactants (e.g., cationic surfactants such as for example dimethyldioctadecyl-ammonium bromide), sugars or sugar derivatives (e.g., cyclodextrin), nucleic acids, and-polymers (e.g., heparin, polyethylene glycol and poloxomer). Surface-altering agents may also include mucolytic agents, e.g., N-acetylcysteine, mugwort, bromelain, papain, clerodendrum, acetylcysteine, bromhexine, carbocisteine, eprazinone, mesna, ambroxol, sobrerol, domiodol, letosteine, stepronin, tiopronin, gelsolin, thymosin β4 dornase alfa, neltenexine, erdosteine, and various DNases including rhDNase. A mucolytic agent or sacrificial agent can be administered separately or concomitantly with a particle, or as a surface-altering agent of the particle (e.g., coated upon, covalently coupled to, co-localized with, or encapsulated within the particle) of the invention to improve transport across a mucosal barrier. Certain agents, e.g., cyclodextrin, may form inclusion complexes with other molecules and can be used to form attachments to additional moieties and facilitate the functionalization of the particle surface and/or the attached molecules or moieties.

Examples of suitable surface-altering agents that are carbohydrates include agar, agarose, alginic acid, amylopectin, amylose, beta-glucan, callose, carrageenan, cellodextrins, cellulin, cellulose, chitin, chitosan, chrysolaminarin, curdlan, cyclodextrin, dextrin, ficoll, fructan, fucoidan, galactomannan, gellan gum, glucan, glucomannan, glycocalyx, glycogen, hemicellulose, hydroxyethyl starch, kefiran, laminarin, mucilage, glycosaminoglycan, natural gum, paramylon, pectin, polysaccharide peptide, schizophyllan, sialyl lewis x, starch, starch gelatinization, sugammadex, xanthan gum, and xyloglucan, as well as fragments and derivatives of such carbohydrates.

Examples of surfactants include but are not limited to L-α-phosphatidylcholine (PC), 1,2-dipalmitoylphosphatidycholine (DPPC), oleic acid, sorbitan trioleate, sorbitan mono-oleate, sorbitan monolaurate, polyoxyethylene (20) sorbitan monolaurate, polyoxyethylene (20) sorbitan monooleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, isopropyl myristate, glyceryl monooleate, glyceryl monostearate, glyceryl monoricinoleate, cetyl alcohol, stearyl alcohol, polyethylene glycol 400, cetyl pyridinium chloride, benzalkonium chloride, olive oil, glyceryl monolaurate, corn oil, cotton seed oil, and sunflower seed oil, lecithin, oleic acid, and sorbitan trioleate.

A pharmaceutically acceptable polymer may be a poly(lactic-co-glycolic) acid (PLGA), poly(D,L-lactic-co-glycolic) acid), polyethylenimine, dioleyltrimethyammoniumpropane/dioleyl-sn-glycerolphosphoethanolamine, polysebacic anhydrides, or other polymers formed from clinically approved monomers. Examples of clinically approved monomers include but are not limited to monomers of sebacic acid and 1,3-bis(carboxyphenoxy)propane.

A pharmaceutically acceptable polymer may be a polyanhydride polymer comprising repeated subunits of Formula A and Formula B, and, optionally, subunits of Formula C, as depicted below: 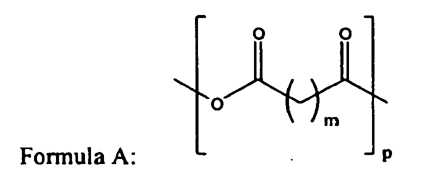 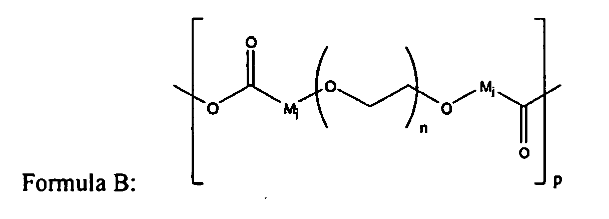 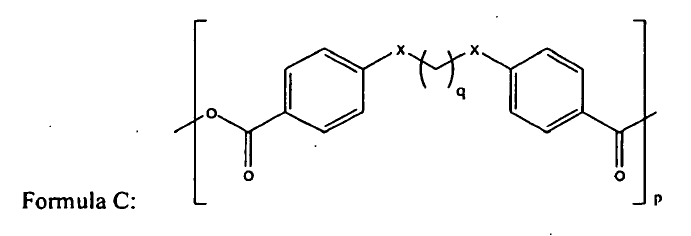 wherein, as valence and stability permit,
M represents, independently for each occurrence, a substituted or unsubstituted methylene, e.g., CH₂, CH(Me), CF₂, CH(OH), C=O, etc., preferably CH₂ or, for an occurrence of M adjacent to O, C=O;
X is absent or, independently for each occurrence, represents a heteroatom selected from NR, O, and S, preferably O;
R represents, independently for each occurrence, H or lower alkyl;
j represents, independently for each occurrence, an integer from 0 to 16, preferably from I to 9;
m represents, independently for each occurrence, an integer from 4 to 20, preferably from 8 to 14, even more preferably 10;
n represents, independently for each occurrence, an integer from 4 to 500, preferably from 10 to 200;
p represents, independently for each occurrence, an integer from 1 to 60, preferably from 4 to 40; and
q represents, independently for each occurrence, an integer from 1 to 20, preferably from 2 to 10, even more preferably from 2 to 6.

In certain embodiments, m, n, and q each, independently, represent a constant value throughout the polymer, i.e., m, n, and q do not vary within a subunit of Formula A, B, or C, or within different subunits of the same formula, within a sample of polymer or a polymer chain.

In certain embodiments, the polymer may contain monomeric units other than those subunits represented by Formulae A, B, and C. In preferred embodiments, however, the polymer consists essentially of subunits of Formulae A, B, and C.

In certain embodiments, a polymer of the present invention has the formula —[K]ₙ—, wherein each occurrence of K represents a subunit of Formula A or B or, optionally, C, as set forth above. Polymer strands may be capped (terminated) with hydroxyl groups (to form carboxylic acids), acyl groups (to form anyhydrides), alkoxy groups (to form esters), or any other suitable capping groups.

In certain embodiments, the subunits of Formula B have a molecular weight between 200 and 1000 daltons, while in other embodiments, the subunits of Formula B have a molecular weight between 4000 and 10,000 daltons. In some embodiments, the subunits of Formula B have molecular weights which vary throughout the polymer between 200 daltons and 10,000 or more daltons, while in other embodiments, the subunits of Formula B have molecular weights that vary only within a narrow range (e.g., 200-300 daltons, or 2,000-3,000 daltons).

In certain embodiments, subunits of Formula B make up between 1 and 80% of the polymer, by weight, preferably between 5 and 60%. In certain embodiments, subunits of, Formula C, if present, may make up between 1% and 80% of the polymer, by weight, preferably between 5 and 60%. In certain embodiments, subunits of Formula A make up between 10% and 99% of the polymer, by weight, preferably between 15% and 95%.

Each subunit may repeat any number of times, and one subunit may occur with substantially the same frequency, more often, or less often than another subunit, such that both subunits may be present in approximately the same amount, or in differing amounts, which may differ slightly or be highly disparate, e.g., one subunit is present nearly to the exclusion of the other.

In certain instances, the polymers are random copolymers, in which the different subunits and/or other monomeric units are distributed randomly throughout the polymer chain. In part, the term "random" is intended to refer to the situation in which the particular distribution or incorporation of monomeric units in a polymer that has more than one type of monomeric unit is not directed or controlled directly by the synthetic protocol, but instead results from features inherent to the polymer system, such as the reactivity, amounts of subunits and other characteristics of the synthetic reaction or other methods of manufacture, processing or treatment.

In certain embodiments, the polymeric chains of such compositions, e.g., which include repetitive elements shown in any of the above formulas, have molecular weights (M_{w}) ranging from about 2000 or less to about 300,000, 600,000 or 1,000,000 or more daltons, or alternatively at least about 10,000, 20,000, 30,000, 40,000, or 50,000 daltons, more particularly at least about 100,000 daltons. Number-average molecular weight (Mₙ) may also vary widely, but generally falls in the range of about 1,000 to about 200,000 daltons, preferably from about 10,000 to about 100,000 daltons and, even more preferably, from about 8,000 to about 50,000 daltons. Most preferably, Mₙ varies between about 12,000 and 45,000 daltons. Within a given sample of a polymer, a wide range of molecular weights may be present. For example, molecules within the sample may have molecular weights that differ by a factor of 2, 5, 10, 20, 50, 100, or more, or that differ from the average molecular weight by a factor of 2, 5, 10, 20, 50, 100, or more.

One method to determine molecular weight is by gel permeation chromatography ("GPC"), e.g., mixed bed columns, CH₂Cl₂ solvent, light scattering detector, and off-line dn/dc. Other methods are known in the art.

Other polymers that may be employed to make the polymeric particles of the invention include but are not limited to cyclodextrin-containing polymers, in particular cationic cyclodextrin-containing polymers, such as those described in U.S. Pat. No. 6,509,323, poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L-lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), polyalkyl cyanoacralate, polyurethane, poly-L-lysine (PLL), hydroxypropyl methacrylate (HPMA), polyethyleneglycol, poly-L-glutamic acid, poly(hydroxy acids), polyanhydrides, polyorthoesters, poly(ester amides), polyamides, poly(ester ethers), polycarbonates, polyalkylenes such as polyethylene and polypropylene, polyalkylene glycols such as poly(ethylene glycol) (PEG), polyalkylene oxides (PEO), polyalkylene terephthalates such as poly(ethylene terephthalate), polyvinyl alcohols (PVA), polyvinyl ethers, polyvinyl esters such as poly(vinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) (jointly referred to herein as "polyacrylic acids"), and copolymers and mixtures thereof, polydioxanone and its copolymers, polyhydroxyalkanoates, poly(propylene fumarate), polyoxymethylene, poloxamers, poly(ortho)esters, poly(butyric acid), poly(valeric acid), poly(lactide-co-caprolactone), trimethylene carbonate, polyvinylpyrrolidone, and the polymers described in Shieh et al., 1994, J. Biomed. Mater. Res., 28, 1465-1475, and in U.S. Patent No. 4,757,128, Hubbell et al., U.S. Pat. Nos. 5,654,381; 5,627,233; 5,628,863; 5,567,440; and 5,567,435. Other suitable polymers include polyorthoesters (e.g. as disclosed in Heller et al., 2000, Eur. J. Pharm. Biopharm., 50:121-128), polyphosphazenes (e.g. as disclosed in Vandorpe et al., 1997, Biomaterials, 18:1147-1152), and polyphosphoesters (e.g. as disclosed in Encyclopedia of Controlled Drug Delivery, pp. 45-60, Ed. E. Mathiowitz, John Wiley & Sons, Inc. New York, 1999) as well as blends and/or block copolymers of two or more such polymers. The carboxyl termini of lactide- and glycolide-containing polymers may optionally be capped, e.g., by esterification, and the hydroxyl termini may optionally be capped, e.g. by etherification or esterification.

Copolymers of two or more polymers described above, including block and/or random copolymers, may also be employed to make the polymeric particles of the invention.

The invention also contemplates employing copolymers of PEG or derivatives thereof (such as units of Formula B, above) with any of the polymers described above to make the polymeric particles of the invention. In certain embodiments, the PEG or derivatives may locate in the interior positions of the copolymer. Alternatively, the PEG or derivatives may locate near or at the terminal positions of the copolymer. In certain embodiments, the microparticles or nanoparticles are formed under conditions that allow regions of PEG to phase separate or otherwise locate to the surface of the particles. While in certain embodiments, the surface-localized PEG regions alone may perform the function of a surface-altering agent, in other embodiments these copolymeric particles comprise an additional surface-altering agent. Such techniques may be applied analogously to form copolymers of other suitable surface-altering agent polymers, such as cyclodextrin-containing polymers, polyanionic polymers, etc.

In certain embodiments, the polymers are soluble in one or more common organic solvents for ease of fabrication and processing. Common organic solvents include such solvents as 2,2,2-trifluoroethanol, chloroform, dichloromethane, dichloroethane, 2-butanone, butyl acetate, ethyl butyrate, acetone, ethyl acetate, dimethylacetamide, N-methyl pyrrolidone, dimethylformamide, and dimethylsulfoxide.

In certain embodiments, the subject particles and compositions include a bioactive agent. A bioactive agent may be a therapeutic agent, a diagnostic agent, or an imaging agent. Examples of therapeutic agents include but are not limited to a nucleic acid or nucleic acid analog (e.g., a DNA or an RNA), a small molecule, a peptidomimetic, a protein, or a combination thereof. In certain embodiments, the diagnostic or imaging agent further comprises a detectable label.

A bioactive agent may be a nucleic acid or analog thereof, e.g., a DNA useful in gene therapy. Alternatively or additionally, an RNA may be employed as a bioactive agent. The RNA may be an RNAi molecule or construct. RNAi refers to "RNA interference," by which expression of a gene or gene product is decreased by introducing into a target cell one or more double-stranded RNAs which are homologous to the gene of interest (particularly to the messenger RNA of the gene of interest). RNAi may also be achieved by introduction of a DNA:RNA complex wherein the antisense strand (relative to the target) is RNA. Either strand may include one or more modifications to the base or sugar-phosphate backbone. Any nucleic acid preparation designed to achieve an RNA interference effect is referred to herein as an siRNA construct.

Alternatively, an antisense nucleic acid is employed as a bioactive agent. An antisense nucleic acid may bind to its target by conventional base pair complementarity, or, for example, in the case of binding to DNA duplexes, through specific interactions in the major groove of the double helix. The antisense oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (*e.g.*, for targeting host cell receptors), or agents facilitating transport across the cell membrane (*see, e.g.,* Letsinger et al., 1989, Proc. Natl. Acad. Sci. U.S.A. 86:6553-6556, Lemaitre et al., 1987, Proc. Natl. Acad. Sci. 84:648-652, PCT Publication No. WO 88/09810, published December 15, 1988) or the blood-brain barrier *(see, e.g.,* PCT Publication No. WO 89/10134, published April 25, 1988), hybridization-triggered cleavage agents *(see, e.g.,* Krol et al., 1988, BioTechniques 6:958- 976) or intercalating agents *(see, e.g.,* Zon, 1988, Pharm. Res. 5:539-549). To this end, the oligonucleotide may be conjugated to another molecule, e.g., a peptide, hybridization triggered cross-linking agent, transport agent, hybridization-triggered cleavage agent, etc.

"Small molecule" as used herein is meant to refer to a molecule having a molecular weight of less than about 3 kDa and most preferably less than about 1.5 kDa. Extensive libraries of chemical and/or biological mixtures comprising arrays of small molecules and/or fungal, bacterial, or algal extracts can be screened with any of the assays known in the art to obtain a desirable bioactive agent for use in or with a particle of the invention.

Peptidomimetics are compounds in which at least a portion of a peptide, such as a therapeutic peptide, is modified, and the three-dimensional structure of the peptidomimetic remains substantially the same as that of the peptide. Peptidomimetics (both peptide and non-peptidyl analogues) may have improved properties (e.g., decreased proteolysis, increased retention or increased bioavailability). Peptidomimetics generally have improved oral availability, which makes them especially suited to treatment of disorders in a human or animal. It should be noted that peptidomimetics may or may not have similar two-dimensional chemical structures, but share common three-dimensional structural features and geometry.

The term "protein," "polypeptide," and "peptide" are used interchangeably herein and generally refer to a polymer formed by at least two amino acids linked via a peptide bond.

Imaging agents (e.g., detectable labels or bioactive agents linked to a detectable label), therapeutic agents, and targeting moieties, such as those described in U.S. Patent Application Publication No. 20030049203 are also contemplated and can be employed with the particles of the present invention.

In certain embodiments, a particle of the invention comprises an imaging agent that may be further attached to a detectable label (e.g., the label can be a radioisotope, fluorescent compound, enzyme or enzyme co-factor). The active moiety may be a radioactive agent, such as: radioactive heavy metals such as iron chelates, radioactive chelates of gadolinium or manganese, positron emitters of oxygen, nitrogen, iron, carbon, or gallium, ⁴³K, ⁵²Fe, ⁵⁷Co, ⁶⁷Cu, ⁶⁷Ga , ⁶⁸Ga, ¹²³I, ¹²⁵I, ¹³¹I, ¹³²I, or ⁹⁹Tc. A particle including such a moiety may be used as an imaging agent and be administered in an amount effective for diagnostic use in a mammal such as a human. In this manner, the localization and accumulation of the imaging agent can be detected. The localization and accumulation of the imaging agent may be detected by radioscintiography, nuclear magnetic resonance imaging, computed tomography, or positron emission tomography. As will be evident to the skilled artisan, the amount of radioisotope to be administered is dependent upon the radioisotope. Those having ordinary skill in the art can readily formulate the amount of the imaging agent to be administered based upon the specific activity and energy of a given radionuclide used as the active moiety. Typically 0.1-100 millicuries per dose of imaging agent, preferably 1-10 millicuries, most often 2-5 millicuries are administered. Thus, compositions according to the present invention useful as imaging agents comprising a targeting moiety conjugated to a radioactive moiety comprise 0.1-100 millicuries, in some embodiments preferably 1-10 millicuries, in some embodiments preferably 2-5 millicuries, in some embodiments more preferably 1-5 millicuries.

The means of detection used to detect the label is dependent of the nature of the label used and the nature of the biological sample used, and may also include fluorescence polarization, high performance liquid chromatography, antibody capture, gel electrophoresis, differential precipitation, organic extraction, size exclusion chromatography, fluorescence microscopy, or fluorescence activated cell sorting (FACS) assay.

In certain embodiments, a bioactive agent or targeting moiety may be covalently coupled to a particle of the invention. In such embodiments, the bioactive agent may preferably be a hydrophilic or charged agent, such that its presence on the surface of the particle increases charge or hydrophilicity of the particle or otherwise increases the particle's mucus resistance. The covalent linkage may be selected to be cleaved under biological conditions, e.g., by chemical or enzymatic hydrolysis or other cleavage processes.

In certain embodiments, a particle of the invention may further comprise a targeting moiety or molecule. The targeting molecule may be covalently linked to any other component of the particle, such as the polymer or a surface-altering agent. The targeting molecule may also be co-localized with a particle, using methods known in the art. The targeting molecule may direct the particle, and thus the included bioactive agent, to a desirable target or location in a patient.

In one embodiment, the targeting moiety is a small molecule. Molecules which may be suitable for use as targeting moieties in the present invention include haptens, epitopes, arid dsDNA fragments and analogs and derivatives thereof. Such moieties bind specifically to antibodies, fragments or analogs thereof, including mimetics (for haptens and epitopes), and zinc finger proteins (for dsDNA fragments). Nutrients believed to trigger receptor-mediated endocytosis and therefore useful targeting moieties include biotin, folate, riboflavin, carnitine, inositol, lipoic acid, niacin, pantothenic acid, thiamin, pyridoxal, ascorbic acid, and the lipid soluble vitamins A, D, E and K. Another exemplary type of small molecule targeting moiety includes steroidal lipids, such as cholesterol, and steroidal hormones, such as estradiol, testosterone, etc.

In another embodiment, the targeting moiety may comprise a protein. Particular types of proteins may be selected based on known characteristics of the target site or target cells. For example, the probe can be an antibody either monoclonal or polyclonal, where a corresponding antigen is displayed at the target site. In situations wherein a certain receptor is expressed by the target cells, the targeting moiety may comprise a protein or peptidomimetic ligand capable of binding to that receptor. Proteins ligands of known cell surface receptors include low density lipoproteins, transferrin, insulin, fibrinolytic enzymes, anti-HER2, platelet binding proteins such as annexins, and biological response modifiers (including interleukin, interferon, erythropoietin and colony-stimulating factor). A number of monoclonal antibodies that bind to a specific type of cell have been developed, including monoclonal antibodies specific for tumor-associated antigens in humans. Among the many such monoclonal antibodies that may be used are anti-TAC, or other interleukin-2 receptor antibodies; 9.2.27 and NR-ML-05 to the 250 kilodalton human melanoma-associated proteoglycan; and NR-LU-10 to a pancarcinoma glycoprotein. An antibody employed in the present invention may be an intact (whole) molecule, a fragment thereof, or a functional equivalent thereof. Examples of antibody fragments are F(ab')₂, Fab', Fab, and Fᵥ fragments, which may be produced by conventional methods or by genetic or protein engineering.

Other preferred targeting moieties include sugars (e.g., glucose, fucose, galactose, mannose) that are recognized by target-specific receptors. For example, instant claimed constructs can be glycosylated with mannose residues (e.g., attached as C-glycosides to a free nitrogen) to yield targeted constructs having higher affinity binding to tumors expressing mannose receptors (e.g., glioblastomas and gangliocytomas), and bacteria, which are also known to express mannose receptors (Bertozzi, C R and M D Bednarski Carbohydrate Research 223:243 (1992); J. Am. Chem. Soc. 114:2242,5543 (1992)), as well as potentially other infectious agents. Certain cells, such as malignant cells and blood cells (e.g., A, AB, B, etc.) display particular carbohydrates, for which a corresponding lectin may serve as a targeting moiety.

Covalent linkage may be effected by various methods known in the art. Moieties, such as surface-altering agents, adhesion-promoting agents, bioactive agents, targeting agents, and other functional moieties discussed herein, to be covalently linked to the surface of a particle (pendant moieties) may be coupled to the surface after formation of the particle, or may be coupled to one or more components prior to formation of the particle, such that, by chance or molecular self-assembly, the moieties locate to the surface of the particle during particle formation, and thus become embedded or enmeshed in the surface of the particle. In certain embodiments, PEG is covalently linked to nanoparticles by reacting a carboxyl group of the particle with an amine group of the PEG, e.g., to form an amide. Moieties may be coupled to the surface of a formed particle in any order or by any attachment that maintains the desired activity of each component, whether in its linked state or following cleavage of a biocleavable linkage, for example. Pendant moieties may be affixed to particles or components by linking functional groups present at the termini of those moieties or components or by linking appropriate functional groups present at any location on either component. Alternatively, the various components may be linked indirectly through a tether molecule as is well known in the art.

Numerous chemical cross-linking methods are known and potentially applicable for conjugating the various portions of the instant constructs. Many known chemical cross-linking methods are non-specific, i.e., they do not direct the point of coupling to any particular site on the molecule. As a result, use of non-specific cross-linking agents may attack functional sites or sterically block active sites, rendering the conjugated molecules inactive.

For coupling simple molecules, it is often possible to control the location of coupling by using protecting groups, functional group-selective reactions, or the differential steric accessibility of particular sites on the molecules. Such strategies are well known to those skilled in the art of chemical synthesis. Protecting groups may include but are not limited to N-terminal protecting groups known in the art of peptide syntheses, including t-butoxy carbonyl (BOC), benzoyl (Bz), fluoren-9-ylmethoxycarbonyl (Fmoc), triphenylmethyl(trityl) and trichloroethoxycarbonxyl (Troc) and the like. The use of various N-protecting groups, e.g., the benzyloxy carbonyl group or the t-butyloxycarbonyl group (Boc), various coupling reagents, e.g., dicyclohexylcarbodiimide (DCC), 1,3-diisopropylcarbodiimide (DIC), 1-(3-dimethylaminopropyl)-3-ethylcarbodiimide (EDC), N-hydroxyazabenzotriazole (HATU), carbonyldiimidazole, or 1-hydroxybenzotriazole monohydrate (HOBT), and various cleavage conditions: for example, trifluoracetic acid (TFA), HCl in dioxane, hydrogenation on Pd-C in organic solvents (such as methanol or ethyl acetate), boron tris(trifluoroacetate), and cyanogen bromide, and reaction in solution with isolation and purification of intermediates are well-known in the art of peptide synthesis, and are equally applicable to the preparation of the subject compounds.

A preferred approach to increasing coupling specificity of complex molecules is direct chemical coupling to a functional group found only once or a few times in one or both of the molecules to be cross-linked. For example, in many proteins, cysteine, which is the only protein amino acid containing a thiol group, occurs only a few times. Also, for example, if a peptide contains no lysine residues, a cross-linking reagent specific for primary amines will be selective for the amino terminus of that peptide. Successful utilization of this approach to increase coupling specificity requires that the molecule have the suitable reactive residues in areas of the molecule that may be altered without loss of the molecule's biological activity.

Coupling of the two constituents can be accomplished via a coupling or conjugating agent. There are several intermolecular cross-linking reagents which can be utilized. *See, e.g.,* Means, G. E. and Feeney, R. E., Chemical Modification of Proteins, Holden-Day, 1974, pp. 39-43. Among these reagents are, for example, J-succinimidyl 3-(2-pyridyldithio) propionate (SPDP) or N,N'-(1,3-phenylene) bismaleimide (both of which are highly specific for sulfhydryl groups and form irreversible linkages); N,N'-ethylene-bis-(iodoacetamide) or other such reagent having 6 to 11 carbon methylene bridges (which relatively specific for sulfhydryl groups); and 1,5-difluoro-2,4-dinitrobenzene (which forms irreversible linkages with amino and tyrosine groups). Other cross-linking reagents useful for this purpose include: p,p'-difluoro-m,m'-dinitrodiphenylsulfone (which forms irreversible cross-linkages with amino and phenolic groups); dimethyl adipimidate (which is specific for amino groups); phenol-1,4-disulfonylchloride (which reacts principally with amino groups); hexamethylenediisocyanate or diisothiocyanate, or azophenyl-p-diisocyanate (which reacts principally with amino groups); glutaraldehyde (which reacts with several different side chains) and disdiazobenzidine (which reacts primarily with tyrosine and histidine).

Cross-linking reagents may be homobifunctional, i.e., having two functional groups that undergo the same reaction. A preferred homobifunctional cross-linking reagent is bismaleimidohexane ("BMH"). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of peptides that contain cysteine residues.

Cross-linking reagents may also be heterobifunctional. Heterobifunctional cross-linking agents have two different functional groups, for example an amine-reactive group and a thiol-reactive group, that will cross-link two proteins having free amines and thiols, respectively. Heterobifunctional cross-linkers provide the ability to design more specific coupling methods for conjugating two chemical entities, thereby reducing the occurrences of unwanted side reactions such as homo-protein polymers. A wide variety of heterobifunctional cross-linkers are known in the art. Examples of heterobifunctional cross-linking agents are succinimidyl 4-(N-maleimidomethyl)-cyclohexane-1- -carboxylate (SMCC), N-succinimidyl (4-iodoacetyl) aminobenzoate (SIAB), 1-ethyl-3-(3-dimethylaminopropyl) carbodiimide hydrochloride (EDC); 4-succinimidyloxycarbonyl- a-methyl-a-(2-pyridyldithio)-tolune (SMPT), N-succinimidyl 3-(2-pyridyldithio) propionate (SPDP), succinimidyl 6-[3-(2-pyridyldithio) propionate] hexanoate (LC-SPDP)succinimidyl 4-(N-maleimidomethyl)-cyclohexane-1-carboxylate (SMCC), m-maleimidobenzoyl-N-hydroxysuccinimide ester (MBS), and succinimide 4-(p-maleimidophenyl)butyrate (SMPB), an extended chain analog of MBS. The succinimidyl group of these cross-linkers reacts with a primary amine, and the thiol-reactive maleimide forms a covalent bond with the thiol of a cysteine residue.

Cross-linking reagents often have low solubility in water. A hydrophilic moiety, such as a sulfonate group, may be added to the cross-linking reagent to improve its water solubility. Sulfo-MBS and sulfo-SMCC are examples of cross-linking reagents modified for water solubility.

Another reactive group useful as part of a heterobifunctional cross-linker is a thiol reactive group. Common thiol-reactive groups include maleimides, halogens, and pyridyl disulfides. Maleimides react specifically with free sulfhydryls (cysteine residues) in minutes, under slightly acidic to neutral (pH 6.5-7.5) conditions. Haloalkyl groups (e.g., iodoacetyl functions) react with thiol groups at physiological pH's. Both of these reactive groups result in the formation of stable thioether bonds.

In addition to the heterobifunctional cross-linkers, there exist a number of other cross-linking agents including homobifunctional and photoreactive cross-linkers. Disuccinimidyl - suberate (DSS), bismaleimidohexane (BMH) and dimethylpimelimidate-2 HCl (DMP) are examples of useful homobifunctional cross-linking agents, and bis-[β-(4-azidosalicylamido)ethyl]disulfide (BASED) and N-succinimidyl-6(4'-azido-2'-nitrophenyl-amino)hexanoate (SANPAH) are examples of useful photoreactive cross-linkers for use in this invention. For a review of protein coupling techniques, see Means et al. (1990) Bioconjugate Chemistry 1:2-12.

Many cross-linking reagents yield a conjugate that is essentially non-cleavable under cellular conditions. However, some cross-linking reagents contain a covalent bond, such as a disulfide, that is cleavable under cellular conditions. For example, dithiobis(succinimidylpropionate) (DSP), Traut's reagent and N-succinimidyl 3-(2-pyridyldithio) propionate (SPDP) are well-known cleavable cross-linkers. The use of a cleavable cross-linking reagent may permit the moiety, such as a therapeutic agent, to separate from the construct after delivery to the target. Direct disulfide linkages may also be useful. Additional cleavable linkages are known in the art and may be employed to advantage in certain embodiments of the present invention.

Many methods for linking compounds, such as proteins, labels, and other chemical entities, to nucleotides are known in the art. Some new cross-linking reagents such as n-maleimidobutyryloxy-succinimide ester (GMBS) and sulfo-GMBS, have reduced immunogenicity. Substituents have been attached to the 5' end of preconstructed oligonucleotides using amidite or H-phosphonate chemistry, as described by Ogilvie, K. K., et al., Pure and Appl Chem (1987) 59:325, and by Froehler, B. C., Nucleic Acids Res (1986) 14:5399. Substituents have also been attached to the 3' end of oligomers, as described by Asseline, U., et al., Tet Lett (1989) 30:2521. This last method utilizes 2,2'-dithioethanol attached to a solid support to displace diisopropylamine from a 3' phosphonate bearing the acridine moiety and is subsequently deleted after oxidation of the phosphorus. Other substituents have been bound to the 3' end of oligomers by alternate methods, including polylysine (Bayard, B., et al., Biochemistry (1986) 25:3730; Lemaitre, M., et al., Nucleosides and Nucleotides (1987) 6:311) and, in addition, disulfides have been used to attach various groups to the 3' terminus, as described by Zuckerman, R., et al., Nucleic Acids Res (1987) 15:5305. It is known that oligonucleotides which are substituted at the 3' end show increased stability and increased resistance to degradation by exonucleases (Lancelot, G., et al., Biochemistry (1985) 24:2521; Asseline, U., et al., Proc Natl Acad Sci USA (1984) 81:3297). Additional methods of attaching non-nucleotide entities to oligonucleotides are discussed in U.S. Pat. Nos. 5,321,131 and 5,414,077.

Alternatively, an oligonucleotide may include one or more modified nucleotides having a group attached via a linker arm to the base. For example, Langer et al (Proc. Natl. Acad. Sci. U.S.A., 78(11):6633-6637, 1981) describes the attachment of biotin to the C-5 position of dUTP by an allylamine linker arm. The attachment of biotin and other groups to the 5-position of pyrimidines via a linker arm is also discussed in U.S. Pat. No. 4,711,955. Nucleotides labeled via a linker arm attached to the 5- or other positions of pyrimidines are also suggested in U.S. Pat. No. 4,948,882. Bisulfite-catalyzed transamination of the N.sup.4-position of cytosine with bifunctional amines is described by Schulman et al. (Nucleic Acids Research, 9(5): 1203-1217, 1981) and Draper et al (Biochemistry, 19: 1774-1781, 1980). By this method, chemical entities are attached via linker arms to cytidine or cytidine-containing polynucleotides. The attachment of biotin to the N4-position of cytidine is disclosed in U.S. Pat. No. 4,828,979 and the linking of moieties to cytidine at the N⁴-position is also set forth in U.S. Pat. Nos. 5,013,831 and 5,241,060. U.S. Pat. No. 5,407,801 describes the preparation of an oligonucleotide triplex wherein a linker arm is conjugated to deoxycytidine via bisulfite-catalyzed transamination. The linker arms include an aminoalkyl or carboxyalkyl linker arm. U.S. Pat. No. 5,405,950 describes cytidine analogs in which a linker arm is attached to the N4-position of the cytosine base.

Numerous cross-linking reagents, including the ones discussed above, are commercially available. Detailed instructions for their use are readily available from the commercial suppliers. A general reference on protein cross-linking and conjugate preparation is: S. S. Wong, Chemistry of Protein Conjugation and Cross-Linking, CRC Press (1991).

Chemical cross-linking may include the use of spacer arms, i.e., linkers or tethers. Spacer arms provide intramolecular flexibility or adjust intramolecular distances between conjugated moieties and thereby may help preserve biological activity. A spacer arm may be in the form of a peptide moiety comprising spacer amino acids. Alternatively, a spacer arm may be part of the cross-linking reagent, such as in "long-chain SPDP" (Pierce Chem. Co., Rockford, III., cat. No. 21651H).

A variety of coupling or crosslinking agents such as protein A, carbodiimide, dimaleimide, dithio-bis-nitrobenzoic acid (DTNB), N-succinimidyl-S-acetyl-thioacetate (SATA), and N-succinimidyl-3-(2-pyrid- yldithio) propionate (SPDP), 6-hydrazinonicotimide (HYNIC), N₃S and N₂S₂ can be used in well-known procedures to synthesize targeted constructs. For example, biotin can be conjugated to an oligonucleotide via DTPA using the bicyclic anhydride method of Hnatowich et al. Int. J. Appl. Radiat. Isotop. 33:327 (1982).

In addition, sulfosuccinimidyl 6-(biotinamido)hexanoate (NHS-LC-biotin, which can be purchased from Pierce Chemical Co. Rockford, III.), "biocytin," a lysine conjugate of biotin, can be useful for making biotin compounds due to the availability of a primary amine. In addition, corresponding biotin acid chloride or acid precursors can be coupled with an amino derivative of the therapeutic agent by known methods. By coupling a biotin moiety to the surface of a particle, another moiety may be coupled to avidin and then coupled to the particle by the strong avidin-biotin affinity, or vice versa.

Analogous methods can also be used to link a surface-altering agent to a small molecule, protein, or other substance in need of such modification.

In certain embodiments where a particle comprises PEG moieties on the surface of the particle, the free hydroxyl group of PEG may be used for linkage or attachment (e.g., covalent attachment) of additional molecules or moieties to the particle.

Imaging labels may be coupled to a particle by covalent bonding directly or indirectly to an atom of the polymer or surface-altering agent, or the label may be non-covalently or covalently associated with the particle through a chelating structure or through an auxiliary molecule such as mannitol, gluconate, glucoheptonate, tartrate, and the like.

Any suitable chelating structure may be used to provide spatial proximity between a radionuclide and the particle through covalent or noncovalent association. Many such chelating structures are known in the art. Preferably, the chelating structure is an N₂S₂ structure, an N₃S structure, an N₄ structure, an isonitrile-containing structure, a hydrazine containing structure, a HYNIC (hydrazinonicotinic acid)-containing structure, a 2-methylthionicotinic acid-containing structure, a carboxylate-containing structure, or the like. In some cases, chelation can be achieved without including a separate chelating structure, because the radionuclide chelates directly to atom(s) in or pendant from the particle, for example to oxygen atoms in the polymer or a polyethylene glycol surface-altering agent.

Radionuclides may be placed in spatial proximity to a particle using known procedures which effect or optimize chelation, association, or attachment of the specific radionuclide to a component of the particle or a moiety pendant from the particle's surface. For example, when ¹²³I is the radionuclide, the imaging agent may be labeled in accordance with the known radioiodination procedures such as direct radioiodination with chloramine T, radioiodination exchange for a halogen or an organometallic group, and the like. When the radionuclide is ⁹⁹mTc, the imaging agent may be labeled using any method suitable for attaching ⁹⁹mTc to a ligand molecule. Preferably, when the radionuclide is ⁹⁹mTc, an auxiliary molecule such as mannitol, gluconate, glucoheptonate, or tartrate is included in the labeling reaction mixture, with or without a chelating structure. More preferably, ⁹⁹mTc is placed in spatial proximity to the targeting molecule by reducing ⁹⁹mTcO₄ with tin in the presence of mannitol and the targeting molecule. Other reducing agents, including tin tartrate or non-tin reductants such as sodium dithionite, may also be used to make an imaging agent according to the invention.

In general, labeling methodologies vary with the choice of radionuclide, the moiety to be labeled and the clinical condition under investigation. Labeling methods using ⁹⁹mTc and ¹¹¹In are described for example in Peters, A. M. et al., Lancet 2: 946-949 (1986); Srivastava, S. C. et al., Semin. Nucl. Med. 14(2):68-82 (1984); Sinn, H. et al., Nucl. Med. (Stuttgart) 13:180, 1984); McAfee, J. G. et al., J. Nucl. Med. 17:480-487, 1976; McAfee, J. G. et al., J. Nucl. Med. 17:480-487, 1976; Welch, M. J. et al., J. Nucl. Med. 18:558-562, 1977; McAfee, J. G., et al., Semin. Nucl. Med. 14(2):83, 1984; Thakur, M. L., et al., Semin. Nucl. Med. 14(2):107, 1984; Danpure, H. J. et al., Br. J. Radiol., 54:597-601, 1981; Danpure, H. J. et al., Br. J. Radiol. 55:247-249, 1982; Peters, A. M. et al., J. Nucl. Med. 24:39-44, 1982; Gunter, K. P. et al., Radiology 149:563-566, 1983; and Thakur, M. L. et al., J. Nucl. Med. 26:518-523, 1985.

Particles can be characterized using standard methods of high field NMR spectra as well as IR, MS, and optical rotation. Elemental analysis, TLC, and/or HPLC can be used as a measure of purity. A purity of at least about 80%, preferably at least about 90%; more preferably at least about 95% and even more preferably at least about 98% is preferred. TLC and/or HPLC can also be used to characterize such compounds.

Once prepared, candidate particles can be screened for ability to carry their bioactive agent(s) across a mucosal barrier. The candidate particles may also be tested for ability to transfect a cell, if the carried bioactive agent is a nucleic acid. In addition, stability of a particle can be tested by incubating the compound in serum, e.g., human serum, and measuring the potential degradation of the compound over time. Stability can also be determined by administering the compound to a subject (human or non-human), obtaining blood samples at various time periods (e.g., 30 min, 1 hour, 24 hours) and analyzing the blood samples for derived or related metabolites.

A "drug," "therapeutic agent," or "medicament," is a biologically, physiologically, or pharmacologically active substance that acts locally or systemically in the human or animal body. A subject composition may include any active substance.

Various forms of the medicaments or drug may be used which are capable of being carried by the particles across mucosal barriers into adjacent tissues or fluids. They may be acidic, basic, or salts. They may be neutral molecules, polar molecules, or molecular complexes capable of hydrogen bonding. They may be in the form of ethers, esters, amides and the like, including prodrugs which are biologically activated when injected into the human or animal body, e.g., by cleavage of an ester or amide. An analgesic agent is also an example of a "medicament." Any additional medicament in a subject composition may vary widely with the purpose for the composition. The term "medicament" includes without limitation, vitamins; mineral supplements; substances used for the treatment, prevention, diagnosis, cure or mitigation of disease or illness; substances which affect the structure or function of the body; or pro-drugs, which become biologically active or more active after they have been placed in a predetermined physiological environment.

Plasticizers and stabilizing agents known in the art may be incorporated in particles of the present invention. In certain embodiments, additives such as plasticizers and stabilizing agents are selected for their biocompatibility. In certain embodiments, the additives are lung surfactants, such as 1,2-dipalmitoylphosphatidycholine (DPPC) and L-α-phosphatidylcholine (PC).

In other embodiments, spheronization enhancers facilitate the production of subject particles that are generally spherical in shape. Substances such as zein, microcrystalline cellulose or microcrystalline cellulose co-processed with sodium carboxymethyl cellulose may confer plasticity to the subject compositions as well as impart strength and integrity. In particular embodiments, during spheronization, extrudates that are rigid, but not plastic, result in the formation of dumbbell shaped particles and/or a high proportion of fines, and extrudates that are plastic, but not rigid, tend to agglomerate and form excessively large particles. In such embodiments, a balance between rigidity and plasticity is desirable. The percent of spheronization enhancer in a formulation typically range from 10 to 90% (w/w). In certain embodiments, a subject composition includes an excipient. A particular excipient may be selected based on its melting point, solubility in a selected solvent (e.g., a solvent that dissolves the polymer and/or the therapeutic agent), and the resulting characteristics of the particles.

Excipients may make up a few percent, about 5%, 10%, 15%, 20%, 25%, 30%, 40%, 50%, or higher percentage of the subject compositions.

Buffers, acids and bases may be incorporated in the subject compositions to adjust their pH. Agents to increase the diffusion distance of agents released from the polymer matrix may also be included.

### 4. Applications: Therapeutic and Diagnostic Compositions

In part, a polymer particle of the present invention includes a biocompatible and preferably biodegradable polymer, such as any polymer discussed above, optionally including any other biocompatible and optionally biodegradable polymer mentioned above or known in the art. The invention provides pharmaceutical compositions that include one or more particles. A pharmaceutical composition may be a therapeutic composition and/or a diagnostic or imaging composition.

### A. Physical Structures of the Subject Compositions

The subject particles, e.g., microparticles or preferably nanoparticles, may comprise polymeric matrices. Microparticles typically comprise a biodegradable polymer matrix and a bioactive agent, e.g., the bioactive agent is encapsulated by or adsorbed to the polymer matrix. Microparticles can be formed by a wide variety of techniques known to those of skill in the art. Examples of microparticle-forming techniques include, but are not limited to, (a) phase separation by emulsification and subsequent organic solvent evaporation (including complex emulsion methods such as oil-in-water emulsions, water-in-oil emulsions, and water-oil-water emulsions); (b) coacervation-phase separation; (c) melt dispersion; (d) interfacial deposition; (e) in situ polymerization; (f) spray-drying and spray-congealing; (g) air suspension coating; and (h) pan and spray coating. These methods, as well as properties and characteristics of microparticles are disclosed in, for example, U.S. Pat. No. 4,652,441; U.S. Pat. No. 5,100,669; U.S. Pat. No. 4,526,938; WO 93/24150; EPA 0258780 A2; U.S. Pat. No. 4,438,253; and U.S. Pat. 5.330.768.

To prepare particles of the present invention, several methods can be employed depending upon the desired application of the delivery vehicles. Suitable methods include, but are not limited to, spray-drying, freeze-drying, air drying, vacuum drying, fluidized-bed drying, milling, co-precipitation and critical fluid extraction. In the case of spray-drying, freeze-drying, air drying, vacuum drying, fluidized-bed drying and critical fluid extraction; the components (stabilizing polyol, bioactive material, buffers, etc.) are first dissolved or suspended in aqueous conditions. In the case of co-precipitation, the components are mixed in organic conditions and processed as described below. Spray-drying can be used to load the particle with the bioactive material. The components are mixed under aqueous conditions and dried using precision nozzles to produce extremely uniform droplets in a drying chamber. Suitable spray drying machines include, but are not limited to, Buchi, NIRO, APV and Lab-plant spray driers used according to the manufacturer's instructions.

The shape of microparticles and nanoparticles may be determined by scanning or transmission electron microscopy. Spherically shaped nanoparticles are used in certain embodiments, e.g., for circulation through the bloodstream. If desired, the particles may be fabricated using known techniques into other shapes that are more useful for a specific application.

In addition to intracellular delivery of a therapeutic agent, it also possible that particles of the subject compositions, such as microparticles or nanoparticles, may undergo endocytosis, thereby obtaining access to the cell. The frequency of such, an endocytosis process will likely depend on the size of any particle.

### B. Dosages and Formulations of the Subject Compositions

In most embodiments, the subject polymers will incorporate the substance to be delivered in an amount sufficient to deliver to a patient a therapeutically effective amount of an incorporated therapeutic agent or other material as part of a diagnostic, prophylactic, or therapeutic treatment. The desired concentration of active compound in the particle will depend on absorption, inactivation, and excretion rates of the drug as well as the delivery rate of the compound from the subject compositions. It is to be noted that dosage values may also vary with the severity of the condition to be alleviated. It is to be further understood that for any particular subject, specific dosage regimens should be adjusted over time according to the individual need and the professional judgment of the person administering or supervising the administration of the compositions. Typically, dosing will be determined using techniques known to one skilled in the art.

Further, the amounts of bioactive substances will vary depending upon the relative potency of the agents selected. Additionally, the optimal concentration and/or quantities or amounts of any particular therapeutic agent may be adjusted to accommodate variations in the treatment parameters. Such treatment parameters include the polymer composition of a particular preparation, the identity of the therapeutic agent utilized, and the clinical use to which the preparation is put, e.g., the site treated, the type of patient, e.g., human or non-human, adult or child, and the nature of the disease or condition.

The concentration and/or amount of any therapeutic agent or other adsorbed or encapsulated material for a given subject composition may readily identified by routine screening in animals, e.g., rats, by screening a range of concentration and/or amounts of the material in question using appropriate assays. Known methods are also available to assay local tissue concentrations, diffusion rates from particles and local blood flow before and after administration of therapeutic formulations according to the invention. One such method is microdialysis, as reviewed by T. E. Robinson et al., 1991, MICRODIALYSIS IN THE NEUROSCIENCES, Techniques, volume 7, Chapter 1. The methods reviewed by Robinson may be applied, in brief, as follows. A microdialysis loop is placed in situ in a test animal. Dialysis fluid is pumped through the loop. When particles according to the invention are injected adjacent to the loop, released drugs are collected in the dialysate in proportion to their local tissue concentrations. The progress of diffusion of the active agents may be determined thereby with suitable calibration procedures using known concentrations of active agents.

In certain embodiments, the dosage of the subject invention may be determined by reference to the plasma concentrations of the therapeutic agent or other encapsulated materials. For example, the maximum plasma concentration (Cₘₐₓ) and the area under the plasma concentration-time curve from time 0 to infinity may be used.

The compositions of the present invention may be administered by various means, depending on their intended use, as is well known in the art. For example, if subject compositions are to be administered orally, it may be formulated as tablets, capsules, granules, powders or syrups. Alternatively, formulations of the present invention may be administered parenterally as injections (intravenous, intramuscular, or subcutaneous), drop infusion preparations, or suppositories. For application by the ophthalmic mucous membrane route, subject compositions may be formulated as eyedrops or eye ointments. These formulations may be prepared by conventional means, and, if desired, the subject compositions may be mixed with any conventional additive, such as a binder, a disintegrating agent, a lubricant, a corrigent, a solubilizing agent, a suspension aid, an emulsifying agent or a coating agent.

In addition, in certain embodiments, subject compositions of the present invention maybe lyophilized or subjected to another appropriate drying technique such as spray drying.

The subject compositions may be administered once, or may be divided into a number of smaller doses to be administered at varying intervals of time, depending in part on the release rate of the compositions and the desired dosage.

Formulations useful in the methods of the present invention include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of a subject composition which may be combined with a carrier material to produce a single dose may vary depending upon the subject being treated, and the particular mode of administration.

Methods of preparing these formulations or compositions include the step of bringing into association subject compositions with the carrier and, optionally, one or more accessory ingredients. In general, the formulations are prepared by uniformly and intimately bringing into association a subject composition with liquid carriers, or finely divided solid carriers, or both, and then, if necessary, shaping the product.

Particles, particularly nanoparticles, which may be administered in inhalant or aerosol formulations according to the invention comprise one or more agents, such as adjuvants, diagnostic agents, imaging agents, or therapeutic agents useful in inhalation therapy.

The particle size of the particulate medicament should be such as to permit inhalation of substantially all of the medicament into the lungs upon administration of the aerosol formulation and will thus desirably be less than 20 microns, preferably in the range 1 to 10 microns, e.g., 1 to 5 microns. The particle size of the medicament may be reduced by conventional means, for example by milling or micronisation.

The final aerosol formulation desirably contains 0.005-90% w/w, preferably 0.005-50%, more preferably 0.005-5% w/w, especially 0.01-1.0% w/w, of medicament relative to the total weight of the formulation.

It is desirable, but by no means required, that the formulations of the invention contain no components which may provoke the degradation of stratospheric ozone. In particular it is desirable that the formulations are substantially free of chlorofluorocarbons such as CCl₃F, CCl₂F₂ and CF₃CCl₃. As used herein "substantially free" means less than 1% w/w based upon the propellant system, in particular less than 0.5%, for example 0.1% or less.

The propellant may optionally contain an adjuvant having a higher polarity and/or a higher boiling point than the propellant. Polar adjuvants which may be used include (e.g., C₂₋₆) aliphatic alcohols and polyols such as ethanol, isopropanol and propylene glycol, preferably ethanol. In general, only small quantities of polar adjuvants (e.g., 0.05-3.0% w/w) may be required to improve the stability of the dispersion--the use of quantities in excess of 5% w/w may tend to dissolve the medicament. Formulations in accordance with the invention may preferably contain less than 1% w/w, e.g., about 0.1% w/w, of polar adjuvant. However, the formulations of the invention are preferably substantially free of polar adjuvants, especially ethanol. Suitable volatile adjuvants include saturated hydrocarbons such as propane, n-butane, isobutane, pentane and isopentane and alkyl ethers such as dimethyl ether. In general, up to 50% w/w of the propellant may comprise a volatile adjuvant, for example 1 to 30% w/w of a volatile saturated C1-C6 hydrocarbon.

Optionally, the aerosol formulations according to the invention may further comprise one or more surfactants. The surfactants must be physiologically acceptable upon administration by inhalation. Within this category are included surfactants such as L-α-phosphatidylcholine (PC), 1,2-dipalmitoylphosphatidycholine (DPPC), oleic acid, sorbitan trioleate, sorbitan mono-oleate, sorbitan monolaurate, polyoxyethylene (20) sorbitan monolaurate, polyoxyethylene (20) sorbitan monooleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, isopropyl myristate, glyceryl monooleate, glyceryl monostearate, glyceryl monoricinoleate, cetyl alcohol, stearyl alcohol, polyethylene glycol 400, cetyl pyridinium chloride, benzalkonium chloride, olive oil, glyceryl monolaurate, corn oil, cotton seed oil, and sunflower seed oil. Preferred surfactants are lecithin, oleic acid, and sorbitan trioleate.

The formulations of the invention may be prepared by dispersal of the particles in the selected propellant and/or co-propellant in an appropriate container, e.g., with the aid of sonication. Preferably, the particles are suspended in co-propellant and filled into a suitable container. The valve of the container is then sealed into place and the propellant introduced by pressure filling through the valve in the conventional manner. The particles may be thus suspended or dissolved in a liquified propellant, sealed in a container with a metering valve and fitted into an actuator. Such metered dose inhalers are well known in the art. The metering valve may meter 10 to 500 µL and preferably 25 to 150 µL. In certain embodiments, dispersal may be achieved using dry powder inhalers (e.g., spinhaler) for the particles (which remain as dry powders). In other embodiments, nanospheres, may be suspended in an aqueous fluid and nebulized into fine droplets to be aerosolized into the lungs.

Sonic nebulizers may be used because they minimize exposing the agent to shear, which may result in degradation of the particles. Ordinarily, an aqueous aerosol is made by formulating an aqueous solution or suspension of the particles together with conventional pharmaceutically acceptable carriers and stabilizers. The carriers and stabilizers vary with the requirements of the particular composition, but typically include non-ionic surfactants (Tweens, Pluronics, or polyethylene glycol), innocuous proteins like serum albumin, sorbitan esters, oleic acid, lecithin, amino acids such as glycine, buffers, salts, sugars, or sugar alcohols. Aerosols generally are prepared from isotonic solutions.

Ophthalmic formulations, eye ointments, powders, solutions and the like, are also contemplated as being within the scope of this invention.

Certain pharmaceutical compositions of this invention suitable for parenteral administration comprise one or more subject compositions in combination with one or more pharmaceutically acceptable sterile, isotonic, aqueous, or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity may be maintained, for example, by the use of coating materials, such as lecithin, by the maintenance of the required particle size in the case of dispersions, and by the use of surfactants.

Microparticle and/or nanoparticle compositions may be suspended in a pharmaceutically acceptable solution, such as saline, Ringer's solution, dextran solution, dextrose solution, sorbitol solution, a solution containing polyvinyl alcohol (from about 1% to about 3%, preferably about 2%), or an osmotically balanced solution comprising a surfactant (such as Tween 80 or Tween 20) and a viscosity-enhancing agent (such as gelatin, alginate, sodium carboxymethylcellulose, etc.). In certain embodiments, the composition is administered subcutaneously. In other embodiments, the composition is administered intravenously. For intravenous delivery, the composition is preferably formulated as microparticles or nanoparticles on average less than about 15 microns, more particularly less than about 10 microns, more particularly less than about 5 microns, and still more particularly less than about 5 microns in average diameter.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia), each containing a predetermined amount of a subject composition as an active ingredient. Subject compositions of the present invention may also be administered as a bolus, electuary, or paste.

In solid dosage forms for oral administration (capsules, tablets, pills, dragees, powders, granules and the like), the subject composition is mixed with one or more pharmaceutically acceptable carriers and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using lactose or milk sugars, as well as high molecular weight polyethylene glycols and the like.

A tablet may be made by compression or molding, optionally with one or more accessory ingredients. Compressed tablets may be prepared using a binder (for example, gelatin or hydroxypropylmethyl cellulose), lubricant, inert diluent, preservative, disintegrant (for example, sodium starch glycolate or cross-linked sodium carboxymethyl cellulose), surface-altering or dispersing agent. Molded tablets may be made by molding in a suitable machine a mixture of the subject composition moistened with an inert liquid diluent. Tablets, and other solid dosage forms, such as dragees, capsules, pills and granules, may optionally be scored or prepared with coatings and shells, such as enteric coatings and other coatings well known in the pharmaceutical-formulating art.

Liquid dosage forms for oral administration include pharmaceutically acceptable emulsions, microemulsions, solutions, suspensions, syrups and elixirs. In addition to the subject compositions, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, corn, peanut, sunflower, soybean, olive, castor, and sesame oils), glycerol, tetrahydrofuryl alcohol, polyethylene glycols and fatty acid esters of sorbitan, and mixtures thereof.

Suspensions, in addition to the subject compositions, may contain suspending agents such as, for example, ethoxylated isostearyl alcohols, polyoxyethylene sorbitol, and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing a subject composition with one or more suitable non-irritating carriers comprising, for example, cocoa butter, polyethylene glycol, a suppository wax, or a salicylate, and which is solid at room temperature, but liquid at body temperature and, therefore, will melt in the appropriate body cavity and release the encapsulated particles. An exemplary formulation for vaginal administration may comprise a bioactive agent that is a contraceptive or an anti-viral, anti-fungal or antibiotic agent.

Formulations which are suitable for vaginal administration also include pessaries, tampons, creams, gels, pastes, foams, or spray formulations containing such carriers as are known in the art to be appropriate.

Dosage forms for transdermal administration include powders, sprays, ointments, pastes, creams, lotions, gels, solutions, patches, and inhalants. A subject composition may be mixed under sterile conditions with a pharmaceutically acceptable carrier, and with any preservatives, buffers, or propellants that may be required. For transdermal administration, the complexes may include lipophilic and hydrophilic groups to achieve the desired water solubility and transport properties.

The ointments, pastes, creams and gels may contain, in addition to subject compositions, other carriers, such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays may contain, in addition to a subject composition, excipients such as lactose, talc, silicic acid, aluminum hydroxide, calcium silicates and polyamide powder, or mixtures of such substances. Sprays may additionally contain customary propellants, such as chlorofluorohydrocarbons and volatile unsubstituted hydrocarbons, such as butane and propane.

### Exemplification

### 1. MATERIALS AND METHODS

### 1.1 Cervicovaginal and Cystic Fibrosis mucus collection and preparation

The cervicovaginal mucus collection procedure was performed as published previously (Boskey, ER, Moench, TR, Hees, PS & Cone, RA (2003) Sexually Transmitted Diseases 30, 107-109, incorporated herein by reference). Collected mucus was used for microscopy within 4 h. The viscosity of fresh samples was observed as a function of shear rate at 37 °C in a Brookfield cone and plate viscometer (Model HADV-III with CP-40 spindle; Brookfield Engineering Lab, Middleboro, MA.).

Human respiratory sputum was expectorated from male and female CF patients (ages 18-35). CF sputum samples from multiple patients were pooled, freeze-dried, and reconstituted in sputum buffer by stirring at 4° C to attain a large volume of homogeneous CF sputum. The volume of sputum buffer added to reconstituted CF sputum samples was determined by mass measurements (the reconstituted CF sputum had the equivalent mass of the fresh CF sputum samples).

### 1.2 Nanoparticle preparation and characterization

100-500 nm yellow-green fluorescent, carboxyl-modified polystyrene (PS) particles (Molecular Probes, Eugene, OR) were covalently modified with diamine PEG (MW ~2kDa; Nektar Therapeutics, San Carlos, CA) via carboxyl-amine reaction in 3:1 excess following manufacturer suggested protocol. Di-amine polyethylene glycol (PEG) of molecular weight 3,400 daltons (Nektar Therapeutics, San Carlos, CA) was dissolved in 50 mM 2-(N-morpholino)ethanesulfonic acid (MES, Sigma, St Louis, MO) buffer at pH 6.0. The use of diamine PEG may result in a free amine group at the end of the surface-bound PEG chains. Yellow-green fluorescent polystyrene nanospheres (Molecular Probes, Eugene, OR) were added to the solution to give final concentrations of 10 mg PEG/ml and 1% solids/ml. The nanospheres had diameters of 100 nm and were carboxyl-modified. Following a 15 min incubation at room temperature, EDAC (1-ethyl-3-(3-dimethylaminopropyl)-carbodiimide) (Sigma, St Louis, MO) was added to the mixture to a concentration of 4 mg/ml. The pH of the solution was adjusted to 6.5 with dilute NaOH and incubated on an orbital shaker for 2 h at room temperature. To quench the reaction, glycine (JT Baker, Phillipsburg, NJ) was added to give a final concentration of 100 mM. The solution was incubated for 30 min at room temperature and subsequently dialyzed extensively against Dulbecco's phosphate-buffered saline (PBS) in a 300,000 kDa MWCO Float-a-lyzer (Spectrum Laboratories, Rancho Dominguez, CA). Unmodified microspheres were dialyzed similarly to remove all traces of sodium azide originally added by the manufacturer.

The size and ξ-potential were determined by dynamic light scattering and laser Doppler anemometry, respectively, using a Zetasizer 3000 (Malvern Instruments, Southborough, MA). Size measurements were performed at 25 °C at a scattering angle of 90°. Samples were diluted in double distilled water and measurements performed according to instrument instructions.

### 1.3 Protein Adsorption to Particles - Measure of PEGylation Effectiveness

To confirm PEG attachment and quantify efficiency in resisting protein adsorption by PEG, 10µL of COOH-particles and PEG-modified particles (~0.04% by mass) were added to 200 µL 0.1 mg/mL rhodamine fluorescent NeutrAvidin (Molecular Probes, Eugene, OR) and incubated on an orbital shaker for 1 hour. Particles were subsequently washed twice in PBS, resuspended to a final concentration of 0.008% by mass, and observed on sealed glass slides/coverslips using a confocal microscope (Zeiss LSM 510, Carl Zeiss Inc., Thornwood, NY) equipped with a 100X/1.4 NA oil-immersion lens. Samples were excited with 488 and 543 lasers, and the pinhole was adjusted to obtain optical slices ranging from less than 0.7-0.8 µm. Identical excitation and detection settings were maintained and all samples were tested sequentially. Particles without avidin incubation served as negative control to ensure negligible bleach over. Maximum pixel intensity for each particle, after conversion to grey scale, was analyzed using SCION Image 4.03b.

### 1.4 Multiple Particle Tracking (MPT) in Cervicovaginal Mucus and CF Mucus

Particle transport rates were measured by analyzing trajectories of fluorescent particles, recorded using a silicon-intensified target camera (VE-1000, Dage-MTI, Michigan, IN) mounted on an inverted epifluorescence microscope equipped with 100X oil-immersion objective (numerical aperture 1.3). Experiments were carried out in 8-well glass chambers (LabTek, Campbell, CA) where diluted particle solutions (0.0082% w/v) were added to 250-500 µL of fresh mucus to a final concentration of 3% v/v (final particle conc 8.25x10⁻⁷ w/v) and incubated for 2 h prior to microscopy. Trajectories of n>100 particles were analyzed for each experiment and three experiments were performed for each condition. Movies were captured with Metamorph software (Universal Imaging Corp.) at a temporal resolution of 66.7 ms for 20 s. The tracking resolution was 10 nm, determined by tracking displacements of particles immobilized with a strong adhesive. The coordinates of nanoparticle centroids were transformed into time-averaged mean squared displacements (MSD), <Δ*r*²(τ)>= [*x*(*t* +τ) - *x*(*t*)]² + [*y*(*t+*τ) *- y(t)*]² (τ= time scale or time lag), from which distributions of MSDs and effective diffusivites were calculated, as previously demonstrated (Dawson, M, Wirtz, D & Hanes, J (2003) Journal of Biological Chemistry 278, 50393-50401., Valentine, MT, Perlman, ZE, Gardel, ML, Shin, JH, Matsudaira, P, Mitchison, TJ & Weitz, DA (2004) Biophys J 86, 4004-14, Mason, TG, Ganesan, K, vanZanten, JH, Wirtz, D & Kuo, SC (1997) Physical Review Letters 79, 3282-3285, all of which are incorporated herein by reference). Additional information for measuring 3D transport by 2D particle tracking is provided in a recent review (Suh, J, Dawson, M & Hanes, J (2005) Adv Drug Deliv Rev 57, 63-78, incorporated herein by reference).

The time-dependent mean square displacements (MSD) of hundreds of PEG-modified 500 nm polystyrene (PS-PEG) particles (0.5% by volume of a 1:20 dilution of 2% particle solution) in CF sputum were determined by multiple particle tracking (MPT). Mucus samples (200 µL) were centrifuged and a portion of the supernatant (40 µL) was replaced with mucolytic solution or PBS to maintain the initial concentration of mucus solids and eliminate any dilution effects. The displacements of particles in the no treatment (PBS) control were identical to that of particles embedded in an unprocessed mucus sample, which was not centrifuged. The tracking resolution, evaluated by tracking 500 nm polystyrene probes in glycerol, was 5 nm.

### 1.5 Particle transport mode classification

The mechanism of particle transport over short and long time scales was classified based on the concept of relative change (RC) of effective diffusivity (D_{eff}). In brief, RC values of particles at short and long time scales were calculated by dividing the D_{eff} of a particle at a probed time scale by the D_{eff} at an earlier reference time scale. By calculating RC values for two time regimes (i.e., short and long time scales), one can obtain the transport mode that describes the particle transport properties over different length and temporal scales. RCₛₕₒᵣₜ was defined at τ_{ref} = 0.2s and τ_{probe} = 1s, whereas RC_{long} was found at reference τ_{ref} = 1s and τ_{probe} = 2s. An RC standard curve, which plots the 95% distribution range of D_{eff} for purely Brownian particles over time scale, was generated based on Monte Carlo simulations and confirmed by tracking polystyrene nanoparticles in glycerol (data not shown). The transport modes of particles that display RC values below the 97.5% range for either short or long time scales were classified as hindered, and the rest were classified as diffusive. Immobile particles are defined as those that display an average MSD smaller than the 10-nm resolution at a time scale of 1s. The rigor of the transport modes classification was confirmed by the slopes of the MSD vs. time scale plots, where diffusive particles possess a slope of approximately 1 and where the slope for hindered particles progressively decrease from 1 with increasing time scale.

### 2. RESULTS AND DISCUSSION

### 2.1 Human cervicovaginal mucus and its rheology.

Cervicovaginal (CV) mucus exhibits macroscopic viscosity within the range (in the higher end) of typical human mucus secretions, including lungs, GI tract, nose, eyes and epididymus. This is partly attributed to the similarity in their chemical composition. For example, the mucin glycoform MUC5B is the major secreted form of mucin in the mucosal layers protecting the CV tract, lungs, nose, and eye. The mucin content, approximately 1-3% by weight, is also similar between cervical, nasal and lung mucus. The composition of water in the aforementioned mucus types all falls within the range of 90-98%.

### 2.2 Real-time transport of COOH-modified nanoparticles

We determined the effect of particle size on transport rates in cervicovaginal (CV) mucus obtained from human volunteers. The hydrodynamic diameters of the particles suspended in water, characterized by dynamic light scattering, are listed in Fig. 8. The addition of uncoated particle at relatively high concentration (2% particles by weight) to CV mucus caused collapse of the mucus fibers into bundles that trapped the particles and prevented their transport (data not shown). However, low concentration of particles (0.008% particles by weight) did not cause bundling and allowed particle movement. As expected, particle transport was highly hindered by the mucus mesh, evident from their low average mean square displacements (MSD) (Fig. 1A). The ensemble-average effective diffusivity (D_{eff}) of COOH-PS particles decreases at short time scales (Fig. 2B), as expected in mucus. By fitting particle MSD versus time scale (τ) to the equation MSD = 4*D*ₒτ^{α}, where *D*ₒ is the diffusion coefficient independent of time scale, one can obtain an average value for α that provides insight into the extent of impediment to particle motion (Note: α = 1 for pure unobstructed Brownian diffusion, such as particles in water). Average α values were 0.16, 0.36 and 0.43 for 100, 200 and 500 nm COOH-PS particles, respectively. Overall, the ensemble-average D_{eff} of 100, 200 and 500 nm COOH-PS particles in mucus (at τ =1s) were reduced by 44000-, 590- and 4600-fold compared to the same particles in water (Fig. 8).

To begin to understand the mechanistic reasons for the unexpectedly low mobility of 100 nm COOH-PS particles (compared to 200 and 500 nm) across all time scales, we sorted particles based on their calculated D_{eff} (at τ=1s) into ten groups (Fig. 1C). Although the fastest 10% of 100 nm COOH-PS particles had roughly similar D_{eff} as compared to 200 and 500 nm COOH-PS particles, the mean D_{eff} values for 200 and 500 nm COOH-PS particles were greater than that for 100 nm COOH-PS particles for all other subgroups (i.e., the slowest 90% of particles), which accounts for the slower ensemble mobility of 100 nm COOH-PS particles. The D_{eff} of individual particles of all sizes spanned a wide range, with the fastest and slowest particles within each particle size differing by at least 4 orders of magnitude (Fig. 1C).

### 2.3 Real-time transport of PEG-modified nanoparticles

Polyethylene glycol (PEG), a hydrophilic and uncharged polymer, was covalently attached to the surface of 100, 200 and 500 nm particles in an attempt to reduce particle interactions with CV mucus. The extent of PEG attachment was comparable for all particles, as shown by their near neutral surface charges and similar efficiencies in resisting adsorption of fluorescently labeled avidin (Fig. 8). PEGylation greatly increased particle transport rates, as evident by the 20, 400- and 1100-fold higher ensemble MSDs (τ = 1s) of 100, 200 and 500 nm PEGylated particles (PEG-PS) compared to corresponding COOH-PS particles of the same size (Fig. 2A). The D_{eff} (τ = 1 s) for 100 nm, 200 nm and 500 nm PEG-PS particles were only reduced by 2000-, 6- and 4-fold compared to that of the expected values for their diffusion in water. The ensemble D_{eff}'s of PEG-PS particles of all three sizes still decreased with increasing time scale (Fig. 2B), but only 100 nm PEG-PS particles experienced extensive obstruction to transport (α = 0.31, 0.81, 0.89 for 100, 200 and 500 nm PEG-PS particles, respectively). PEGylation not only reduced impediment for larger PEG-PS particles (200 and 500 nm), but also increased the homogeneity of transport compared to similar sized COOH-PS particles (Fig. 2C).

The greatly improved transport rates upon PEGylation, especially for larger particles, were largely due to a marked reduction in the fraction of mucoadhesive (immobile + hindered) particles (Fig. 2D & 2E). Indeed, 2 kDa PEG increased the fraction of mucus-penetrating (diffusive) particles to nearly 70% (Fig. 2F). This directly demonstrates that non-adhesive nanoparticles larger than the previously reported upper limit of theoretical mesh size of mucus (200 nm) can undergo rapid transport in human mucus.

### 2.4 Properties of particles coated with high M W. (10 kDa) PEG

High MW PEG is widely used as a mucoadhesive agent (Bures, P., Y. Huang, E. Oral, and N.A. Peppas, Surface modifications and molecular imprinting of polymers in medical and pharmaceutical applications. J Control Release, 2001. 72(1-3): p. 25-33, Huang, Y., W. Leobandung, A. Foss, and N.A. Peppas, Molecular aspects of muco- and-bioadhesion: tethered structures and site-specific surfaces. J Control Release, 2000. 65(1-2): p. 63-71., Lele, B.S. and A.S. Hoffman, Mucoadhesive drug carriers based on complexes of poly(acrylic acid) and PEGylated drugs having hydrolysable PEG-anhydride-drug linkages. J Control Release, 2000. 69(2): p. 237-48., Peppas, N.A., K.B. Keys, M. Torres-Lugo, and A.M. Lowman, Poly(ethylene glycol)-containing hydrogels in drug delivery. J Control Release, 1999. 62(1-2): p. 81-7.). To test its effect as a coating for nanoparticles, 10kDa PEG was covalently attached to the surface of 200 nm particles (PEG_{10kDa}-PS). In sharp contrast to the PEG_{2kDa}-PS counterparts, particles having a dense coating of 10 kDa PEG showed greatly reduced particle transport rates in fresh human CV mucus, as evident by the 2300-fold lower ensemble MSDs (τ = 1s) compared to particles modified with 2 kDa PEG (Fig. 3A). In fact, the extensive obstruction to transport for PEG_{10kDa}-PS resulted in an ensemble MSD (τ = Is) nearly 6-fold lower than that for similar-sized COOH-PS particles, due in large part to the high fractions of both immobile and strongly hindered particles (i.e. *mucoadhesive*) (Fig. 3B). Without wishing to be bound by theory, it is possible that low MW PEG eliminates mucoadhesion by minimizing both hydrogen bonding and interpenetration of PEG chains into the mucus gel, while higher MW PEG, with longer, flexible chains that extend farther from the surface of the particle, penetrates into the mucus gel in a fashion that impedes diffusion. Alternative approaches to modifying particles with high MW PEG, however, may control the length and flexibility of pendant PEG chains, thereby providing a mucus-resistant surface property.

### 2.5 N-Acetyl Cysteine Improves Manoparticle Transport in Human CF Sputum.

Mucus degrading agents, such as rhDNase (which hydrolyzes linear DNA) and N-acetylcysteine (NAC) (which cleaves disulphide and sulphahydryl bonds present in mucin), are used clinically to increase the rate of mucus clearance (Hanes, J., M. Dawson, Y. Har-el, J. Suh, and J. Fiegel, Gene Delivery to the Lung. Pharmaceutical Inhalation Aerosol Technology, A.J.Hickey, Editor. Marcel Dekker Inc.: New York, 2003: p. 489-539.). These agents may also be valuable adjuvants in increasing the rate of nanoparticle transport in mucus (Ferrari, S., C. Kitson, R. Farley, R. Steel, C. Marriott, D.A. Parkins, M. Scarpa, B. Wainwright, M.J. Evans, W.H. Colledge, D.M. Geddes, and E.W. Alton, Mucus altering agents as adjuncts for nonviral gene transfer to airway epithelium. Gene Ther, 2001. 8(18): p. 1380-6). Previously, we quantified the effect of rhDNase on particle transport rates in CF mucus using multiple particle tracking (Fig. 4). The distribution of individual particle transport rates was remarkably more homogeneous at 30 mins post-treatment with rhDNAse than in the no treatment control (compare Fig. 4A and 4B). However, despite the reduction in bulk viscoeleastic properties by more than 50% (Fig. 4C), treatment with rhDNase actually *reduced* the overall ensemble averaged transport rates of nanoparticles (Fig. 4D). Alternative approaches to treating mucus with rhDNAse, for example different incubation times and different buffers, may improve its utility as a mucolytic agent. In contrast, treatment with NAC significantly improved the transport rates of nanoparticles (Fig. 4E).

Ensemble geometric mean square displacements show that pretreatment of mucus with neutralized N-acetyl-L-cysteine increased transport rates 10.7-fold compared to no-treatment control (Fig. 5A). Classifying the trajectories of particle motion into different transport modes (immobile, hindered, diffusive) show that the diffusive fraction of 500 nm PEG-PS is enhanced 3-fold compared to the no-treatment control (Fig. 5B).

### 2.6 Particle Trajectories

The typical trajectories of particles undergoing transport in CV mucus were recorded and quantified by microscopy. Particles fall into three general categories: immobile (Fig 6A), hindered (Fig 6B), and diffusive (Fig 6C).

### 2.7 Quantification of PEG surface coating

Rapid transport by polymeric nanoparticles in undiluted human mucus is likely a direct consequence of improved surface coating of PEG. Previously, 500 nm PEG coated particles (as disclosed in Example 6B in WO 2005/072710 A2), with a low PEG density (Prep A, Fig 7), were found to improve transport ~10-fold compared to uncoated particles of similar size. In contrast, higher density of surface PEG (Prep B, Fig 7) was able to mediate improvements in transport of 500 nm particles by up to ~1100-fold compared to similar sized uncoated counterparts. This directly underscores the importance of high density of surface PEG coating in dictating particle transport in mucus.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. A particle comprising an outer surface and one or more surface-altering moieties disposed on the outer surface that reduce mucoadhesion of the particle, wherein
(a) the mass of the surface-altering moiety makes up at least 1/3400 of the mass of the particle, and/or
(b) the surface-altering moiety is present on the outer surface at a density of greater than 0.01 units per nanometer squared.

2. The particle of claim 1 wherein the particle comprises a core, and the surface-altering moiety is
(a) covalently attached to the core, or
(b) non-covalently adsorbed to the core.

3. The particle of any preceding claim, wherein the particle comprises a core that comprises a polymer, optionally wherein the polymer comprises surface-altering moieties disposed on the outer surface.

4. The particle of any preceding claim, wherein one or more bioactive agents are associated with the particle.

5. The particle of any preceding claim, wherein said particle has a particle size of greater than 100 nm, 200 nm, 300 nm, 400 nm, or 500 nm.

6. The particle of claim 4, wherein the bioactive agent is an imaging agent or a therapeutic agent.

7. The particle of any preceding claim, wherein the surface-altering moiety is
(a) hydrophilic, or
(b) is a surfactant.

8. The particle of any preceding claim, wherein the particle comprises a copolymer comprising PEG or a derivative of PEG and wherein the PEG or derivatives are located near or at the terminal positions of the copolymer.

9. The particle of any preceding claim, wherein the core and/or the surface-altering moiety comprises PEG.

10. The particle of claim 8 or 9, wherein the PEG has a molecular weight of 1 kDa, 2 kDa, 3 kDa, 4 kDa, 6 kDa, or 8 kDa.

11. The particle of any preceding claim, wherein the surface-altering moiety comprises a block copolymer of oxyethylene and oxypropylene.

12. The particle of any preceding claim, wherein the surface-altering moiety has a density of at least 0.02, of at least 0.05, of at least 0.1, of at least 0.2, of at least 0.5, of at least 1, of at least 2, of at least 5, of at least 10, or of at least 20 units per nanometer squared.

13. The particle of any preceding claim, wherein the particle has a zeta potential between -10 mV and 10 mV, between -10 mV and 5 mV, between -5 mV and 5 mv, or between -2 mV and 2mV.

14. A pharmaceutical composition comprising the particle of any preceding claim and one or more pharmaceutically acceptable carriers.

15. An inhaler comprising the particle of any of claims 1-13.

16. A pharmaceutical preparation suitable for
(i) inhalation
(ii) injection, or
(iii) topical administration to a mucus membrane,
comprising a bioactive agent associated with a surface-altering moiety, wherein
(a) the mass of the surface-altering moiety makes up at least 1/3400 of the mass of the bioactive agent; and/or
(b) the surface-altering moiety is present on the outer surface of the bioactive agent at a density of greater than 0.01 units per nanometer squared.

17. An enveloped virus comprising a surface-altering moiety, wherein said virus diffuses through human cervicovaginal mucus at a diffusivity at a time scale of 1 second that is more than 20-fold greater than the diffusivity at which a corresponding virus lacking the surface-altering moiety diffuses through human cervicovaginal mucus, and wherein
(a) the mass of the surface-altering moiety makes up at least 1/3400 of the mass of the virus; and/or
(b) the surface-altering moiety is present on the outer surface of the virus at a density of greater than 0.01 units per nanometer squared.

## Patentansprüche

1. Partikel, das eine äußere Oberfläche und eine oder mehrere oberflächenverändernde Einheiten umfasst, die auf der äußeren Oberfläche angeordnet sind und die die Mucoadhäsion des Partikles vermindern, wobei
(a) die Masse der oberflächenverändernden Einheit bis zu 1/3400 der Masse des Partikels ausmacht, und/oder
(b) die oberflächenverändernde Einheit auf der äußeren Oberfläche in einer Dichte von größer als 0,01 Einheiten pro Quadratnanometer vorkommt.

2. Partikel nach Anspruch 1, wobei das Partikel einen Kern umfasst, und die oberflächenverändernde Einheit
(a) kovalent an den Kern angeheftet ist, oder
(b) nicht kovalent an den Kern adsorbiert ist.

3. Partikel nach einem der vorherigen Ansprüche, wobei das Partikel einen Kern umfasst, der ein Polymer umfasst, wobei das Polymer gegebenenfalls oberflächenverändernde Einheiten umfasst, die auf der äußeren Oberfläche angeordnet sind.

4. Partikel nach einem der vorherigen Ansprüche, wobei ein oder mehrere bioaktive Wirkstoffe mit dem Partikel assoziiert sind.

5. Partikel nach einem der vorherigen Ansprüche, wobei das Partikel eine Partikelgröße von größer als 100nm, 200nm, 300nm, 400nm, oder 500nm hat.

6. Partikel nach Anspruch 4, wobei der bioaktive Wirkstoff ein bildgebender Wirkstoff oder ein therapeutischer Wirkstoff ist.

7. Partikel nach einem der vorherigen Ansprüche, wobei die oberflächenverändernde Einheit
(a) hydrophil ist, oder
(b) ein oberflächenaktiver Stoff ist.

8. Partikel nach einem der vorherigen Ansprüche, wobei das Partikel ein Copolymer umfasst, das PEG oder ein Derivat von PEG umfasst und wobei das PEG oder das Derivat nahe oder an den terminalen Positionen des Copolymers sitzt/sitzten.

9. Partikel nach einem der vorherigen Ansprüche, wobei der Kern und/oder die oberflächenverändernde Einheit PEG umfasst.

10. Partikel nach Anspruch 8 oder 9, wobei das PEG ein Molekulargewicht von 1 kDa, 2 kDa, 3 kDa, 4 kDa, 6 kDa oder 8 kDa hat.

11. Partikel nach einem der vorherigen Ansprüche, wobei die oberflächenverändernde Einheit ein Blockpolymer oder Oxyethylen und Oxypropylen umfasst.

12. Partikel nach einem der vorherigen Ansprüche, wobei die oberflächenverändernde Einheit eine Dichte von mindestens 0,02, von mindestens 0,05, von mindestens 0,1, von mindestens 0,2, von mindestens 0,5, von mindestens 1, von mindestens 2, von mindestens 5, von mindestens 10 oder von mindestens 20 Einheiten pro Quadratnanometer aufweist.

13. Partikel nach einem der vorherigen Ansprüche, wobei das Partikel ein Zetapotential zwischen -10mV und 10mV, zwischen -10mV und 5mV, zwischen -5mV und 5mV oder zwischen -2mV und 2mV aufweist.

14. Pharmazeutische Zusammensetzung, die das Partikel nach einem der vorherigen Ansprüche und einen oder mehrere pharmazeutisch verträgliche Träger umfasst.

15. Inhalator, der das Partikel nach einem der Ansprüche 1-13 umfasst.

16. Pharmazeutisches Präparat, das zur
(i) Inhalation,
(ii) Injektion, oder
(iii) topischen Verabreichung auf die Schleimhaut
geeignet ist und einen bioaktiven Wirkstoff umfasst, der mit einer oberflächenverändernden Einheit assoziiert ist, wobei
(a) die Masse der oberflächenverändernden Einheit mindestens 1/3400 der Masse des bioaktiven Wirkstoffs ausmacht, und/oder
(b) die oberflächenverändernde Einheit auf der äußeren Oberfläche des bioaktiven Wirkstoffs in einer Dichte von größer als 0,01 Einheiten pro Quadratnanometer vorkommt.

17. Eingehülltes Virus, das eine oberflächenverändernde Einheit umfasst, wobei das Virus durch menschlichen cervicovaginalen Schleim mit einer Diffusität in einem Zeitmaßstab von 1 Sekunde diffundiert, die mehr als 20-fach größer als die Diffusität ist, mit der ein entsprechendes Virus, dem die oberflächenverändernde Einheit fehlt, durch menschlichen cervicovaginalen Schleim diffundiert, und wobei
(a) die Masse der oberflächenverändernden Einheit mindestes 1/3400 der Masse des Virus ausmacht, und/oder
(b) die oberflächenverändernde Einheit auf der äußeren Oberfläche des Virus in einer Dichte von größer als 0,01 Einheiten pro Quadratnanometer vorkommt.

## Revendications

1. Particule comprenant une surface externe et un ou plusieurs fragments modificateurs de surface placés sur la surface externe qui réduisent la mucoadhésion de la particule, dans laquelle
(a) la masse du fragment modificateur de surface représente au moins 1/3400 de la masse de la particule, et/ou
(b) le fragment modificateur de surface est présent sur la surface externe à une densité supérieure à 0,01 unité par nanomètre carré.

2. Particule selon la revendication 1, dans laquelle la particule comprend un noyau, et le fragment modificateur de surface est
(a) attaché de manière covalente au noyau, ou
(b) absorbé de manière non covalente dans le noyau.

3. Particule selon l'une quelconque des revendications précédentes, dans laquelle la particule comprend un noyau qui comprend un polymère, le polymère comprenant facultativement des fragments modificateurs de surface placés sur la surface externe.

4. Particule selon l'une quelconque des revendications précédentes, dans laquelle un ou plusieurs agents bioactifs sont associés à la particule.

5. Particule selon l'une quelconque des revendications précédentes, dans laquelle ladite particule possède une taille de particule supérieure à 100 nm, 200 nm, 300 nm, 400 nm ou 500 nm.

6. Particule selon la revendication 4, dans laquelle l'agent bioactif est un agent d'imagerie ou un agent thérapeutique.

7. Particule selon l'une quelconque des revendications précédentes, dans laquelle le fragment modificateur de surface est
(a) hydrophile, ou
(b) est un tensioactif.

8. Particule selon l'une quelconque des revendications précédentes, dans laquelle la particule comprend un copolymère comprenant un PEG ou un dérivé de PEG et dans laquelle le PEG ou ses dérivés sont situés à proximité ou au niveau des positions terminales du copolymère.

9. Particule selon l'une quelconque des revendications précédentes, dans laquelle le noyau et/ou le fragment modificateur de surface comprend du PEG.

10. Particule selon la revendication 8 ou 9, dans laquelle le PEG possède un poids moléculaire de 1 kDa, 2 kDa, 3 kDa, 4 kDa, 6 kDa ou 8 kDa.

11. Particule selon l'une quelconque des revendications précédentes, dans laquelle le fragment modificateur de surface comprend un copolymère bloc d'oxyéthylène ou oxypropylène.

12. Particule selon l'une quelconque des revendications précédentes, dans laquelle le fragment modificateur de surface possède une densité d'au moins 0,02, d'au moins 0,05, d'au moins 0,1, d'au moins 0,2, d'au moins 0,5, d'au moins 1, d'au moins 2, d'au moins 5, d'au moins 10 ou d'au moins 20 unités par nanomètre carré.

13. Particule selon l'une quelconque des revendications précédentes, dans laquelle la particule possède un potentiel zêta compris entre -10 mV et 10 mV, entre -10 mV et 5 mV, entre -5 mV et 5 mV ou entre -2 mV et 2 mV.

14. Composition pharmaceutique comprenant la particule selon l'une quelconque des revendications précédentes et un ou plusieurs excipients pharmaceutiquement acceptables.

15. Inhalateur comprenant la particule selon l'une quelconque des revendications 1-13.

16. Préparation pharmaceutique appropriée pour
(i) une inhalation,
(ii) une injection, ou
(iii) une administration topique sur une membrane muqueuse, comprenant un agent bioactif associé à un fragment modificateur de surface, dans laquelle
(a) la masse du fragment modificateur de surface représente au moins 1/3400 de la masse de l'agent bioactif , et/ou
(b) le fragment modificateur de surface est présent sur la surface externe de l'agent bioactif à une densité supérieure à 0,01 unité par nanomètre carré.

17. Virus enveloppé comprenant un fragment modificateur de surface, dans lequel ledit virus diffuse à travers la muqueuse cervicovaginale humaine à une diffusivité sur une échelle de temps de 1 seconde, ce qui est plus de 20 fois supérieure à la diffusivité à laquelle un virus correspondant sans fragment modificateur de surface diffuse à travers la muqueuse cervicovaginale humaine, et dans lequel
(a) la masse du fragment modificateur de surface représente au moins 1/3400 de la masse du virus , et/ou
(b) le fragment modificateur de surface est présent sur la surface externe du virus à une densité supérieure à 0,01 unité par nanomètre carré.
